(19) Europäisches Patentamt
European Patent Office
Office européen des brevets

(11) EP 1 061 964 B2

(12) NEW EUROPEAN PATENT SPECIFICATION
After opposition procedure

(45) Date of publication and mention of the opposition decision:
**28.10.2009 Bulletin 2009/44**

(45) Mention of the grant of the patent:
**16.02.2005 Bulletin 2005/07**

(21) Application number: **99912447.2**

(22) Date of filing: **11.03.1999**

(51) Int Cl.:
***A61L 15/46*** *(2006.01)*

(86) International application number:
**PCT/US1999/005396**

(87) International publication number:
**WO 1999/045976 (16.09.1999 Gazette 1999/37)**

# (54) PROTON DONATING ACTIVES IN ABSORBENT ARTICLES

PROTONENDONATOREN IN SAUGKÖRPERN

AGENTS ACTIFS DONNEURS DE PROTONS CONTENUS DANS DES ARTICLES ABSORBANTS

(84) Designated Contracting States:
**AT BE CH CY DE DK ES FI FR GB GR IE IT LI LU NL PT SE**

(30) Priority: **12.03.1998 US 41509**

(43) Date of publication of application:
**27.12.2000 Bulletin 2000/52**

(73) Proprietor: **THE PROCTER & GAMBLE COMPANY**
**Cincinnati, Ohio 45202 (US)**

(72) Inventors:
- **McOSKER, Jocelyn, Elaine**
  **Loveland, OH 45140 (US)**
- **ROE, Donald, Carroll**
  **West Chester, OH 45069 (US)**
- **FRANXMAN, James, Joseph**
  **Cincinnati, OH 45251 (US)**
- **KLOFTA, Thomas, James**
  **Cincinnati, OH 45249 (US)**
- **BERG, Ronald, Wayne**
  **Fairfield, OH 45014 (US)**
- **WARREN, Raphael**
  **Cincinnati, OH 45237 (US)**

(74) Representative: **L'Huillier, Florent Charles et al**
**Procter & Gamble Service GmbH**
**Sulzbacher Strasse 40-50**
**65824 Schwalbach am Taunus (DE)**

(56) References cited:

| | |
|---|---|
| **WO-A1-96//16681** | **WO-A1-96//16682** |
| **WO-A2-97//05908** | **WO-A2-97//46205** |
| **DE-A1- 4 136 540** | **DE-A1- 4 243 119** |
| **US-A- 4 382 919** | **US-A- 4 556 560** |
| **US-A- 4 685 909** | **US-A- 5 525 346** |

- **'BioChemika Ultra', 05 October 2005, SIGMA ALDRICH COM., INTERNET article HTTP.//WWW.SIGMAALDRICH.COM/BRANDS/ FLUKA_RI EDEL_HOME/BIOSC.: 'Extract of Biological Buffers'**



Printed by Jouve, 75001 PARIS (FR)

**Description**

BACKGROUND OF THE INVENTION

The invention relates to absorbent articles such as diapers, training pants, adult incontinence briefs, feminine hygiene products, and the like, that comprise proton donating active(s) useful for the prevention and treatment of diaper rash and diaper dermatitis. More particularly, a skin care composition which comprises at least one proton donating active selected from the group consisting of polyacrylic acid and partially neutralized polyacrylic acid is disposed on the absorbent article. Such proton donating actives function by helping maintain skin pH in a slightly acidic condition after insult by bodily exudates, thus reducing the redness and inflammation that may result therefrom.

Diaper rash is a common form of irritation and inflammation of those parts of an infant's body normally covered by a diaper. This condition is also referred to as diaper dermatitis, napkin dermatitis, napkin rash and nappy rash. While certainly more common in infants, this condition is not, in fact, limited to infants. Any individual who suffers from incontinence to the extent that the use of absorbent articles is required may develop this condition. This ranges from newborns, to the elderly, to critically ill or nonambulatory individuals.

Many types of disposable absorbent products, such as diapers, incontinence pads, incontinence briefs, sanitary napkins, panty liners, and the like, are available that have a high capacity for absorbing urine and other body exudates. Disposable products of this type generally comprise some sort of liquid-pervious topsheet material, an absorbent core, and a liquid-impervious backsheet material. Although these types of absorbent structures may be highly efficient for the absorption of liquids, it is well recognized that long-term wear of such absorbent articles may compromise the underlying skin in terms of overhydration or exposure to skin irritants commonly found in body exudates. Section 333.503 of Title 21 of the Code of Federal Regulations defines diaper rash as "[a]n inflammatory skin condition in the diaper area (perineum, buttocks, lower abdomen, and inner thighs) caused by one or more of the following factors: moisture, occlusion, chafing, continued contact with urine or feces or both, or mechanical or chemical irritation." It is generally accepted by the medical profession that true diaper rash or diaper dermatitis is a condition which is, in its most simple stages, a contact irritant dermatitis resulting from extended contact of the skin with urine, or feces, or both. While it is known that body waste "causes" diaper rash, the precise component or components of the urine or feces which are responsible for the resulting irritation of the skin have not been conclusively identified. It is most likely that the causes are multifactorial in nature. Among the most commonly accepted factors linked to diaper rash are ammonia, fecal rash are ammonia, fecal enzymes, bacteria, the products of bacterial action, urine pH, and *Candida albicans.* Overhydration of the skin caused by exposure of the skin to urine, high humidity, or occlusive contact of the skin with absorbent articles is also effective in diminishing the barrier properties of skin, thereby enhancing the susceptibility of the skin to enzymes and to invasion by *Candida albicans.*

In view of the foregoing proposed causes of diaper rash, many approaches have been taken in an attempt to reduce or prevent its occurrence. Many of the most practical approaches attempt to address multiple causes or important cofactors. Reducing skin hydration by frequent changing of diapers, the use of moisture absorbing powders, the use of superabsorbent materials, and improving air flow in diapers are well known approaches. The use of artificial barriers is also widely practiced. Typical of these is the use of a topical cream, ointment, lotion or paste to provide some degree of physical barrier protection to the skin against fecal or urine irritants, regardless of their specific nature. However, the barrier approach, while reducing access of irritants to the skin, may be occlusive in itself and can be aesthetically unpleasant.

It is further known that one cause of diaper dermatitis or "diaper rash" is free ammonia generated in a urine-wet diaper in contact with an infant's skin. The ammonia is formed by bacterial enzymatic decomposition of urinary urea by a wide variety of fecal bacteria such as *Bacterium ammoniagenes,* a saprophytic gram positive bacillus, and *Proteus vulgaris,* a gram negative bacillus. In view of this knowledge, the art has proceeded along essentially two lines in the prevention of diaper rash. In one approach, the prior art has attempted to prevent the liberation of ammonia from urine-wet diapers by means of chemical agents which trap the gaseous ammonia generated by the ammonia producing bacteria. Such ammonia immobilizing agents include inter alia weak organic and inorganic acids such as acetic, citric, and boric acid, capable of forming ammonium salts. The ammonia immobilizing agent may be impregnated throughout the absorptive wadding or located in discrete gas permeable sachets enfolded within the diaper.

Another approach to the prevention of diaper rash has been to incorporate a bacteriostatic agent in the diaper structure. Various carboxylic acids have been used for this purpose. U.S. Patent No. 3,707,148, issued to Bryce on December 26, 1972, for example, describes the use of carboxylic acids to inhibit enzymatic activity and ammonia formation. This patent discloses disposable diaper structures impregnated with citric, malic, maleic, malonic, succinic, tartaric, and fumaric acids. However, several of the specified carboxylic acids, citric acid for example, dissolve too rapidly when the diaper is wetted with urine, such that the urine as it wicks out to the edge of the diaper is relatively concentrated in acid, and the center of the diaper loses its protection against ammonia formation. As a result, skin irritation can occur at the diaper margins due to the excessively low pH at this point. Other carboxylic acids, e.g., fumaric acid, while dissolving

more slowly relative to wicking rate, still produce excessively low pH at the edge of the diaper because they are relatively strong acids and there are no basic ions for buffer due to ion exchange of the urine as it wicks through the diaper. U.S. Patent 3,964,486, issued to Blaney on June 22, 1976 describes one solution to this problem; providing a relatively weak acid having a slow dissolution rate relative to the wicking rate of urine in a diaper. Specifically, the Blaney patent indicates that adipic acid is ideally suited for such purposes. Similarly, U.S. Patent 4,685,909, issued to Berg, et al. on August 11, 1987 describes absorbent articles with pH control agents (i.e. organic and inorganic acids) and hydrogel in discrete zones within the absorbent article. However, because such acids depend on absorption of urine by the diaper or other absorbent article, their effectiveness may be limited. For example, maintaining a wearer's skin at an acidic pH using this approach is potentially difficult given that there is frequently free urine (e. g. not absorbed by the absorbent article) and feces present adjacent to the wearer's skin surface. In such a situation, one must rely on the ability of skin to maintain a free hydrogen ion concentration that effectively counteracts basic ions present in such bodily exudates. A limitation of this approach, therefore, is that in order to be effective, the capacity of the skin to resist changes in pH due to free urine and feces must be increased by the application of pH control agents before the skin actually comes into contact with the feces, ideally without disturbing the skin's own homeostatic pH balance.

Diapers impregnated with skin lotions comprising a buffer system are also known. For example, U.S. Patent 5,525,346, issued to Hartung, et al. on June 11, 1996 describes oil-in-water emulsions comprising a non-ionic emulsifier, a dimethicone, sodium citrate, citric acid, aloe vera, preservatives, and water. The patent further describes diapers where at least the portion of the diaper that contacts a wearer's genitals and buttocks is impregnated with the oil-in-water emulsion. While such diapers may provide a skin care benefit, impregnating the diaper with such an emulsion can substantially interfere with the functionality of the diaper. For example, the portion of a diaper contacting a wearer's genitals and buttocks is also the portion of the diaper that primarily receives bodily exudates, particularly urine. If this receiving area is impregnated with an emulsion as is described in U.S. Patent 5,525,346, the absorptive properties thereof will be substantially compromised leading to an increased risk of leakage. Further, given the aqueous continuous phase of the skin lotion described in U.S. Patent 5,525,346, the absorbent elements of the diaper may draw the aqueous phase away from the surface reducing any skin care benefit provided by the lotion.

Thus, there is a continuing need to counteract the effects of ammonia and fecal enzymes on the skin of those wearing absorbent articles. There is a further need to provide improved absorbent articles that help maintain the pH of a wearer's skin in its natural acidic state more specifically, between about 4.0 and about 6.0

## SUMMARY OF THE INVENTION

The Applicants have discovered that it is possible to help maintain an acidic skin pH by the use of proton donating actives, according to claim 1 that transfer from an absorbent article to a wearer's skin. Particularly preferred materials are proton donating actives that are effective in reducing the ability of fecal enzymes to decompose urinary urea into ammonia in addition to neutralizing any ammonia that may be present adjacent to a wearer's skin. A particularly preferred means of delivering such ingredients is to incorporate them into a skin care composition that transfers from the absorbent article to a wearer's skin carrying such proton donating actives to the surface of a wearer's skin.

## BRIEF DESCRIPTION OF THE DRAWINGS

While the specification concludes with claims particularly pointing out and distinctly claiming the present invention, it is believed that the present invention will be better understood from the following description in conjunction with the following drawings, in which like reference numbers identify identical elements and wherein:

Figure 1 is a plan view of an absorbent article of the present invention in the form of a diaper.

Figure 2a is a side view showing placement of the skin analog used in the lotion transfer test.

Figure 2b is a plan view showing placement of the skin analog used in the lotion transfer test.

## DETAILED DESCRIPTION OF THE INVENTION

### I. Definitions

As used herein, the term "comprising" means that the various components, ingredients, or steps can be conjointly employed in practicing the present invention. Accordingly, the term "comprising" encompasses the more restrictive terms "consisting essentially of" and "consisting of".

As used herein a "proton donating active" is a substance or mixture of substances, that, when the substance

or mixture of substances is incorporated into a skin care composition and applied to a subject's skin as is described in the Skin pH Reduction Test in the TEST METHODS section below, causes a decrease in skin pH.

As used herein, "an absorbent article comprising a proton donating active" means any absorbent article, such as those described below, typically having a liquid impervious backsheet, a liquid pervious topsheet, and an absorbent core positioned between the topsheet and the backsheet, wherein the proton donating active is positioned within or on the topsheet, and/or within or on the absorbent core, and/or within or on the backsheet, and/or within or on any other portion of the absorbent article, including cuffs, side panels, fasteners, and the like.

As used herein, the term "skin care composition comprising a proton donating active" refers to any composition employed to transfer a minimum effective amount of the proton donating active from an absorbent article to a wearer's skin to provide a therapeutic and/or protective skin benefit for the treatment or prevention of skin conditions, such as diaper dermatitis. Representative materials are discussed in detail below.

As used herein, the term "wearer-contacting surface" of an absorbent article is one or more surfaces of any article components that may contact the wearer's body at some time during the wear period. Body contacting surfaces include, but are not limited to, portions of the topsheet, leg cuffs, waist region, side panels, and the like, which may contact a wearer during use.

Other terms are defined herein where initially discussed.

With respect to proton donating active (s) and/or a skin care composition (s), all percentages, ratios and proportions used herein are by weight unless otherwise specified.

II. Absorbent Articles

As used herein, the term "absorbent article" refers to a device which absorbs and retains body exudates. The term "disposable" is used herein to describe absorbent articles which are not intended to be laundered or otherwise restored or reused as an absorbent article after a single use. Examples of disposable absorbent articles include feminine hygiene garments such as sanitary napkins, panty liners and tampons, diapers, incontinence briefs, diaper holders, training pants, and the like.

Disposable absorbent articles typically comprise a liquid pervious topsheet, a liquid impervious backsheet and an absorbent core positioned between the topsheet and the backsheet. Disposable absorbent articles and components thereof, including the topsheet, backsheet, absorbent core, and any individual layers of these components, have a body facing surface and a garment facing surface. As used herein "body facing surface" means that surface of the article or component which is intended to be worn toward or adjacent to the body of the wearer, while the "garment facing surface" is on the opposite side and is intended to be worn toward or placed adjacent to the wearer's clothing or undergarments when the disposable absorbent article is worn.

The following description generally discusses the absorbent core, topsheet, and backsheet materials that are useful in disposable absorbent articles. It is to be understood that this general description applies to these components of the specific absorbent articles shown in Figure 1 and further described below, in addition to those of other disposable absorbent articles which are generally described herein.

In general, the absorbent core is capable of absorbing or retaining liquids (e.g., menses, urine, and/or other body exudates). The absorbent core is preferably compressible, conformable, and non-irritating to the wearer's skin. The absorbent core may be manufactured in a wide variety of sizes and shapes (e.g., rectangular, oval, hourglass, "T" shaped, dog bone, asymmetric, etc.). In addition to absorbent composites, the absorbent core may include any of a wide variety of liquid-absorbent materials commonly used in absorbent articles, such as comminuted wood pulp, which is generally referred to as airfelt. Examples of other suitable absorbent materials for use in the absorbent core include creped cellulose wadding; meltblown polymers including coform; chemically stiffened, modified or cross-linked cellulosic fibers; synthetic fibers such as crimped polyester fibers; peat moss; tissue, including tissue wraps and tissue laminates; absorbent foams; absorbent sponges; superabsorbent polymers; absorbent gelling materials; or any equivalent material or combinations of materials, or mixtures of these.

The configuration and construction of the absorbent core may be varied (e.g., the absorbent core may have varying caliper zones and/or have a profile so as to be thicker in the center; hydrophilic gradients; gradients of absorbent composites; superabsorbent gradients; or lower average density and lower average basis weight zones, e.g., acquisition zones; or may comprise one or more layers or structures). The total absorbent capacity of the absorbent core should however, be compatible with the design loading and the intended use of the absorbent article. Further, the size and absorbent capacity of the absorbent core may be varied to accommodate different uses such as diapers, incontinence pads, panty liners, regular sanitary napkins, and overnight sanitary napkins, and to accommodate wearers ranging from infants to adults. The absorbent core can also include other absorbent components that are often used in absorbent articles, for example, a dusting layer, a wicking or acquisition layer, or a secondary topsheet for increasing the wearer's comfort.

The topsheet is preferably compliant, soft feeling, and non-irritating to the wearer's skin. Further, the topsheet

is liquid pervious, permitting liquids (e.g., menses and/or urine) to readily penetrate through its thickness. A suitable topsheet may be manufactured from a wide range of materials such as woven and nonwoven materials (e.g., a nonwoven web of fibers), including apertured nonwovens; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers. When the topsheet comprises a nonwoven web, the web may be manufactured by a wide number of known techniques. For example, the web may be spunbonded, spunlace carded, wet-laid, melt-blown, hydroentangled, hydroformed, hydroapertured, combinations of the above, or the like. Whether comprised of a woven or nonwoven material, the topsheet preferably comprises skin care compositions comprising proton donating active(s), as described further below.

The backsheet is impervious to liquids (e.g., menses and/or urine) and preferably comprises a thin plastic film, although other flexible liquid impervious materials may also be used. As used herein, the term "flexible" refers to materials which are compliant and will readily conform to the general shape and contours of the human body. The backsheet prevents the exudates absorbed and contained in the absorbent core from wetting articles which contact the absorbent article such as bedsheets, pants, pajamas and undergarments. The backsheet may thus comprise a woven or nonwoven material, polymeric films such as thermoplastic films of polyethylene or polypropylene, or composite materials such as a film-coated nonwoven material. A suitable backsheet is a polyethylene film having a thickness of from about 0.012 mm (0.5 mil) to about 0.051 mm. (2.0 mils). Exemplary polyethylene films are manufactured by Clopay Corporation of Cincinnati, OH, under the designation P18-1401 and by Tredegar Film Products of Terre Haute, IN, under the designation XP-39385. The backsheet is preferably embossed and/or matte finished to provide a more clothlike appearance. Further, the backsheet may permit vapors to escape from the absorbent core (i.e., the backsheet is breathable) while still preventing exudates from passing through the backsheet. The size of the backsheet is dictated by the size of the absorbent core and the exact absorbent article design selected.

The backsheet and the topsheet are positioned adjacent the garment facing surface and the body facing surface, respectively, of the absorbent core. The absorbent core is preferably joined with the topsheet, the backsheet, or both in any manner as is known by attachment means (not shown in Figure 1) such as those well known in the art. However, embodiments of the absorbent articles are envisioned wherein portions or the entire absorbent core are unattached to either the topsheet, the backsheet, or both.

For example, the backsheet and/or the topsheet may be secured to the absorbent core or to each other by a uniform continuous layer of adhesive, a patterned layer of adhesive, or an array of separate lines, spirals, or spots of adhesive. Adhesives which have been found to be satisfactory are manufactured by H.B. Fuller Company of St. Paul, MN under the designation HL-1258 or H-2031. The attachment means will preferably comprise an open pattern network of filaments of adhesive as is disclosed in U.S. Patent 4,573,986, issued to Minetola, et al. on March 4, 1986. An exemplary attachment means of an open pattern network of filaments comprises several lines of adhesive filaments swirled into a spiral pattern as illustrated by the apparatus and method shown in U.S. Patent 3,911,173, issued to Sprague, Jr. on October 7, 1975; U.S. Patent 4,785,996, issued to Zwieker, et al. on November 22, 1978; and U.S. Patent 4,842,666, issued to Werenicz on June 27, 1989. Alternatively, the attachment means may comprise heat bonds, pressure bonds, ultrasonic bonds, dynamic mechanical bonds, or any other suitable attachment means or combinations of these attachment means as are known in the art.

Preferred disposable absorbent article of the invention, having proton donating active(s) or proton donating active-containing compositions incorporated therein and, more preferably, having a body contacting surface treated with a proton donating active-containing composition, are diapers. As used herein, the term "diaper" refers to an absorbent article generally worn by infants, and incontinent persons, that is worn about the lower torso of the wearer. In other words, the term "diaper" includes infant diapers, training pants, adult incontinence devices, etc.

Figure 1 is a plan view of the diaper 50 useful in the invention in its flat-out, uncontracted state (i.e., with elastic induced contraction pulled out) with portions of the structure being cut-away to more clearly show the construction of the diaper 50 and with the portion of the diaper 50 which faces away from the wearer (the outer surface) oriented towards the viewer. As shown in Figure 1, the diaper 50 preferably comprises a liquid pervious topsheet 520, a liquid impervious backsheet 530 joined with the topsheet 520, an absorbent core 540 positioned between the topsheet 520 and the backsheet 530, the absorbent core 540 having a garment facing surface 542, a body facing surface 544, side edges 546, waist edges 548, and ears 549. The diaper 50 preferably further comprises elasticized leg cuffs 550, and elastic waist feature multiply designed as 560, and a fastening system generally multiply designed as 570.

The diaper 50 is shown in Figure 1 to have an outer surface 52, an inner surface 54 corresponding to the body facing surface which is opposed to the outer surface 52, a first waist region 56, a second waist region 58, and a periphery 51 which is defined by the outer edges of the diaper 50 in which the longitudinal edges are designated 55 and the end edges are designated 57. (While the skilled artisan will recognize that a diaper is usually described in terms of having a pair of waist regions and a crotch region between the waist regions, in this application, for simplicity of terminology,

the diaper 50 is described as having only waist regions including a portion of the diaper which would typically be designated as part of the crotch region). The body facing surface 54 of the diaper 50 comprises that portion of the diaper 50 which is positioned adjacent to the wearer's body during use. The body facing surface 54 generally is formed by at least a portion of the topsheet 520 and other components that may be joined to the topsheet 520, such as leg cuffs 550, as well as any regions to which the topsheet may not extend but which still contact the wearer, such as the waist feature 560, side panels, and the like. The outer surface 52 comprises that portion of the diaper 50 which is positioned away from the wearer's body (i.e., the outer surface 52 generally is formed by at least a portion of the backsheet 530 and other components that may be joined to the backsheet 530). The first waist region 56 and the second waist region 58 extend, respectively, from the end edges 57 of the periphery 51 to the lateral centerline 53 of the diaper 50. Figure 1 also shows the longitudinal centerline 59.

Figure 1 shows a preferred embodiment of the diaper 50 in which the topsheet 520 and the backsheet 530 have length and width dimensions generally larger than those of the absorbent core 540. The elasticized leg cuffs 550 and the backsheet 530 extend beyond the edges of the absorbent core 540 to thereby form the periphery 51 of the diaper 50.

Diapers of the present invention can have a number of well known configurations, with the absorbent cores thereof being adapted to the present invention. Exemplary configurations are described generally in U.S. Patent 3,860,003, issued to Buell on January 14, 1975; U.S. Patent 5,151,092, issued to Buell et al. on September 29, 1992; U.S. Patent 5,221,274 issued to Buell et al. on June 22, 1993. Another diaper configuration to which the present invention can be readily adapted is described in U.S. Patent 5,554,145 issued to Roe, et al., on September 10, 1996. The absorbent cores of diapers described in this patent can be adapted in light of the teachings herein to include an absorbent composite as an absorbent gelling material described therein.

A topsheet 520 which is particularly suitable for use in the diaper 50, is carded and thermally bonded by means well known to those skilled in the fabrics art. A satisfactory topsheet for the present invention comprises staple length polypropylene fibers having a denier of about 2.2. As used herein, the term "staple length fibers" refers to those fibers having a length of at least about 15.9 mm (0.625 inches). Preferably, the topsheet has a basis weight from about 14 to about 25 grams per square meter. A suitable topsheet is manufactured by Veratec, Inc., a Division of International Paper Company, of Walpole, MA under the designation P-8.

The topsheet 520 of diaper 50 is preferably made of a hydrophilic material to promote rapid transfer of liquids (e.g., urine) through the topsheet. If the topsheet is made of a hydrophobic material, at least portions of the upper surface of the topsheet are treated to be hydrophilic so that liquids will transfer through the topsheet more rapidly. This diminishes the likelihood that body exudates will flow off the topsheet rather than being drawn through the topsheet and being absorbed by the absorbent core. The topsheet can be rendered hydrophilic by treating it with a surfactant. Suitable methods for treating the topsheet with a surfactant include spraying the topsheet material with the surfactant and immersing the material into the surfactant. A more detailed discussion of such a treatment and hydrophilicity is contained in U.S. Patents 4,988,344 issued to Reising, et al on January 29, 1991 and U.S. Patent 4,988,345 issued to Reising on January 29, 1991.

Alternatively, the topsheet may be in the form of an apertured formed film, which is preferred in feminine hygiene absorbent articles. Apertured formed films are useful because they are pervious to body liquids and yet non-absorbent and have a reduced tendency to allow liquids to pass back through and rewet the wearer's skin. Thus, the surface of the formed film that is in contact with the body remains dry, thereby reducing body soiling and creating a more comfortable feel for the wearer. Suitable formed films are described in U.S. Patent 3,929,135, issued to Thompson on December 30, 1975; U.S. Patent 4,324,246, issued to Mullane, et al on April 13, 1982; U.S. Patent 4,342,314, issued to Radel. et al. on August 3, 1982; U.S. Patent 4,463,045, issued to Ahr et al. on July 31, 1984; and U.S. 5,006,394, issued to Baird on April 9, 1991. Particularly preferred microapertured formed film topsheets are disclosed in U.S. Patent 4,609,518, issued to Curro et at on September 2, 1986 and U.S. Patent 4,629,643, issued to Curro et al on December 16, 1986. The preferred topsheet for use in feminine hygiene products is the formed film described in one or more of the above patents and marketed on sanitary napkins by The Procter & Gamble Co. of Cincinnati, OH as "DRI-WEAVE®."

The body facing surface of the formed film topsheet can be hydrophilic so as to help body liquids to transfer through the topsheet faster than if the body surface was not hydrophilic so as to diminish the likelihood that liquid will flow off the topsheet rather than flowing into and being absorbed by the absorbent structure. In a preferred embodiment, surfactant is incorporated into the polymeric materials of the formed film topsheet such as is described in U.S. Statutory Invention Registration H1670, published on July 1, 1997 in the name of Aziz, et al.. Alternatively, the body facing surface of the topsheet can be made hydrophilic by treating it with a surfactant such as is described in the above referenced U.S. 4,950,264.

In a preferred embodiment of a diaper as described herein, the backsheet 530 has a modified hourglass shape extending beyond the absorbent core a minimum distance of about 1.3 cm to about 6.4 cm (about 0.5 to about 2.5 inch) around the entire diaper periphery.

The absorbent core 540 may take on any size or shape that is compatible with the diaper 50. One preferred

embodiment of the diaper 50 has an asymmetric, modified T-shaped absorbent core 540 having ears in the first waist region but a generally rectangular shape in the second waist region. Exemplary absorbent materials for use as the absorbent core of articles useful in the present methods are described, e.g., in U.S. Patent 4,610,678 issued to Weisman et al. on September 9, 1986; U.S. Patent 4,673,402 issued to Weisman et al. on June 16, 1987; U.S. Patent 4,888,231 issued to Angstadt on December 19, 1989; and U.S. Patent 4,834,735, issued to Alemany et al. on May 30, 1989. The absorbent core may further comprise the dual core system containing an acquisition/distribution core of chemically stiffened fibers positioned over an absorbent storage core as detailed in U.S. Patent 5,234,423, issued to Alemany et al., on August 10, 1993; and in U.S. Patent 5,147,345, issued to Young, LaVon and Taylor on September 15, 1992.

In a preferred embodiment, the diaper 50 further comprises elasticized leg cuffs 550 for providing improved containment of liquids and other body exudates; an elastic waist feature 560 that provides improved fit and containment; and a fastening system 570 which forms a side closure which maintains the first waist region 56 and the second waist region 58 in an overlapping configuration such that lateral tensions are maintained around the circumference of the diaper to maintain the diaper on the wearer. The diaper 50 may also comprise elasticized waist bands (not shown) and/or elasticized side panels (also not shown) in the waist regions 56 and 58 to provide an elastically extensible feature that provides a more comfortable and contouring fit and more effective application of the diaper 50.

The elasticized leg cuffs 550 can be constructed in a number of different configurations, including those described in the above-mentioned U.S. Patent No. 3,860,003; U.S. Patent No. 4,909,803, issued to Aziz et al. on Mar. 20, 1990; U.S. Patent No. 4,695,278, issued to Lawson on Sep. 22, 1987; and U.S. Patent No. 4,795,454, issued to Dragoo on Jan. 3, 1989. Absorbent articles having elasticized cuffs that are treated with a composition that may be useful herein are disclosed in copending U.S. Patent application Serial Nos, 08/766,386 and 08/840,039, filed December 3, 1996 and April 24, 1997.

The elasticized waist feature preferably comprises an elasticized waistband (not shown) that may be constructed in a number of different configurations including those described in U.S. Patent No. 4,515,595 issued to Kievit et al. on May 7, 1985; U.S. Patent No. 5,026,364 issued to Robertson on Jun. 25, 1991; and the above referenced U.S. Patent No. 5,151,092 issued to Buell et al. on Sep. 29, 1992.

The elasticized side panels may be constructed in a number of configurations. Examples of diapers with elasticized side panels positioned in the ears (ear flaps) of the diaper are disclosed in U.S. Patent No. 4,857,067, issued to Wood, et al. on Aug. 15, 1989; U.S. Patent No. 4,381,781, issued to Sciaraffa, et al. on May 3, 1983; U.S. Patent No. 4,938,753, issued to Van Gompel, et al. on Jul. 3, 1990; and U.S. Patent No. 5,151,092, issued to Buell et al. on Sep. 29, 1992.

Exemplary fastening systems 570 are disclosed in U.S. Patent No. 4,846,815, issued to Scripps on Jul. 11, 1989; U.S. Patent No. 4,894,060, issued to Nestegard on Jan. 16, 1990; U.S. Patent No. 4,946,527, issued to Battrell on Aug. 7, 1990; U.S. Patent No. 3,848,594, issued to Buell on Nov. 19, 1974; U.S. Patent No. B1 4,662,875, issued to Hirotsu, et al. on May 5, 1987; and U.S. Patent No. 5,151,092, issued to Buell, et al. on Sep. 29, 1992.

The diaper 50 is preferably applied to a wearer by positioning one of the waist regions of the diaper, preferably the second waist region 58, under the wearer's back and drawing the remainder of the diaper between the wearer's legs so that the other waist region, preferably the first waist region 56, is positioned across the front of the wearer. The fastening system 570 is then applied to effect a side closure.

In alternative embodiments (not shown) of the present invention, the absorbent article may be provided with means for improving contact between the topsheet and a wearer's skin. In one embodiment, the absorbent article can be provided with elastic means, as described in U.S. Patent 4,892,536 issued in the name of DesMarais, et al. on January 9, 1990, in U. S. Patent 4, 990,147, issued in the name of Freeland on February 5, 1991, and in U.S. Patent application Serial No. 07.993,198, filed in the name of Freeland, et al. on December 18, 1992, which lift the topsheet to improve contact with a wearer's perianal region. In another embodiment, described in U.S. Patent 5,171,236, issued in the name of Dreier, et al. on December 15, 1992, a diaper is provided with spacing means to lift the topsheet. In yet another embodiment, described in U.S. Statutory Invention Registration H1687, published in the name of Roe, et al. on October 7, 1997, the absorbent article is provided with a gluteal blocking device which lifts the topsheet into a wearer's gluteal groove.

Of course, it will be recognized that any absorbent article design that is capable of incorporating a proton donating active into a delivery system, as described below, may be utilized in the present invention. The disclosure above is merely for illustrative purposes.

The present invention may also employ training pants as an absorbent article wherein the training pants are also provided with a composition comprising a proton donating active(s). The term "training pants", as used herein, refers to disposable garments having fixed sides and leg openings designed for infant or adults wearers. Training pants (also referred in the art as "pull on" products) are placed in position on the wearer by inserting the wearer's legs into the leg openings and sliding the training pant into position about the wearer's lower torso. Suitable training pants are disclosed in U.S. Patent No. 5,246,433, issued to Hasse, et al. on September 21, 1993; U.S. Patent No. 5,569,234, issued to Buell, et al. on October 29, 1996; U.S. Patent No. 4,940,464, issued to Van Compel et al. on July 10, 1990; and U.S. Patent

No. 5,092,861, issued to Nomura et al. on March 3, 1992.

Another disposable absorbent article for use in the present invention is an incontinence article. The term "incontinence article" refers to pads, undergarments (pads held in place by a suspension system of some type, such as a belt, or the like), inserts for absorbent articles, capacity boosters for absorbent articles, briefs, bed pads, and the like regardless of whether they are worn by adults or other incontinent persons. Suitable incontinence articles are disclosed in U.S. Patent No. 4,253,461 issued to Strickland, et al. on March 3, 1981; U.S. Patent Nos. 4,597,760 and 4,597,761 issued to Buell on July 1, 1986; the above-mentioned U.S. Patent No. 4,704,115; U.S. Patent No. 4,909,802 issued to Ahr, et al. on April 16, 1987; U.S. Patent No. 4,964,860 issued to Gipson, et al. on October 23, 1990; and in U.S. Patent 5,304,16 1, issued in the name of Noel, et al. on April 19, 1994.

Another disposable absorbent article for use in the present invention is a feminine hygiene article, such as a sanitary napkin. Suitable feminine hygiene articles are disclosed in U.S. Patent No. 4,556,146, issued to Swanson et al. on December 3, 1985; U.S. Patent No. B14,589,876, issued to Van Tilberg on April 27, 1993; U.S. Patent No. 4,687,478, issued to Van Tilburg on August 18, 1997; U.S. Patent No. 4,950,264, issued to Osborn, III on August 21, 1990; U.S. Patent No. 5,009,653, issued to Osborn, III on April 23, 1991; U.S. Patent 5,267,992, issued to Van Tilburg on December 7, 1993; U.S. Patent No. 5,389,094, issued to Lavash et al. on February 14, 1995; U.S. Patent No. 5,413,568; issued to Roach et al. on May 9, 1995; U.S. Patent No. 5,460,623, issued to Emenaker et al. on October 24, 1995; U.S. Patent No. 5,489,283, issued Van Tilburg on February 6, 1996; U.S. Patent No. 5,569,231, issued to Emenaker et al. on October 29, 1996; and U.S. Patent No. 5,620,430, issued to Bamber on April 15, 1997.

III. <u>Proton Donating Actives</u>

As noted above, the proton donating actives can help maintain a wearer's skin at its natural acidic pH. For example such proton donating actives can be effective in neutralizing any high pH (i.e. >7) components of bodily exudates. Chemically suitable proton donating actives are effective in helping maintain skin pH in at least a slightly acidic condition. As used herein, a material is "effective in helping maintain acidic skin pH" if it is effective in extending the time a subject's skin remains at an acidic pH when tested according to the Skin pH Reduction Test described in the TEST METHODS section below. Further, such materials are, of necessity, pharmaceutically acceptable. As used herein, a material is "pharmaceutically acceptable" if the material may be used in the manner of this invention without undue adverse side effects, such as toxicity, irritation, or allergic response, commensurate with a reasonable benefit/risk ratio.

The skin care composition comprises at least one proton donating active selected from the group consisting of polyacrylic acid and partially neutralized polyacrylic acid.

In order that the proton donating active be effective in helping maintain skin at an acidic pH the proton donating active should be provided at a level of at least 0.01% of the skin care composition; typically at least about 0.5%; preferably at least about 3%. Such suitable proton donating actives may be used as a component of the skin care compositions of the present invention at a level of between about 0.01% and about 40%, depending on the specific proton donating active chosen. Preferably, the proton donating actives are used at a level of between about 0.5% and about 20% of the skin care composition of the present invention. In particularly preferred embodiments of the present invention, the proton donating actives are provided at a level of between about 3% and about 7%.

IV. <u>Delivery Systems</u>

A proton donating active delivery system in an absorbent article preferably contains a minimum effective concentration of a proton donating active(s) and does not itself interfere with the normal function of the various structures of the article (e.g., the absorbency of the core, the liquid perviousness of the top sheet, and the like). In a preferred embodiment, described in greater detail below, the proton donating active(s) is positioned in proximity to the wearer's skin in a clean dry article, such as on or near a body contacting surface, from where at least a portion of the proton donating active(s) may be delivered to the wearer's skin during normal use of the article, preferably before a bowel movement occurs, for availability to act at the skin/feces interface after a bowel movement. In a more preferred embodiment, the proton donating active(s) is (are) a component of a skin care composition containing various emollients and immobilizing agents, as described further below, that is delivered directly from a wearer-contacting surface to the wearer's skin to perform a barrier function to high pH exudates, as well as helping maintain the wearer's skin at an acidic pH. Most preferably, repeated normal use of articles having delivery systems that deliver proton donating actives directly to the skin provides an accumulation of the proton donating active for more effective prevention of inflammation of the skin due to contact with urine and feces.

Thus, proton donating actives, or compositions containing them, may be incorporated into absorbent articles in any delivery system known to those skilled in the art that facilitates contact of a proton donating active with a wearer's skin to inhibit pH increases thereon. The delivery system may be a component of any portion or portions of the absorbent article including, but not limited to, the topsheet, the absorbent core, the backsheet, other secondary structures such as

additional sheets, specialized structures to contain bowel movements (e.g., bowel movement "pockets"), and the like, and any cuffs, side panels, fasteners and the like that may also be a component of the article, whether or not the portion of the article is a wearer-contacting surface. Such delivery systems include those which deliver the proton donating active in dispersed form as a component of another composition, in solution in a substantially anhydrous composition, and in an aqueous vehicle as a part of a substantially oleaginous composition (e.g. as a water-in-oil emulsion). Exemplary delivery systems include, but are not limited to, those described below.

The delivery system may provide the proton donating active in powder, flake or particulate form. The delivery system may include pressure-rupturable or dissolvable microcapsules or "bubbles" containing the proton donating active or a proton donating active-containing composition. Further, the delivery system may include the ingredients or an ingredient-containing composition in any other form that is activated (e.g. by hydrolysis of a precursor material) in the presence of bodily exudates. A particularly suitable delivery system for use with the proton donating actives of the present invention is one having a continuous phase comprising substantially oleaginous materials. As used herein a delivery system comprises "substantially oleaginous materials" if at least about fifty percent of the continuous phase thereof comprises oleaginous emollients, immobilizing agents, and the like as are discussed individually below. Dispersions or solutions of solid proton donating actives in substantially anhydrous compositions and water-in-oil emulsions, wherein the proton donating actives are a component in the disperse aqueous phase thereof, are specifically contemplated as having a continuous phase comprising substantially oleaginous materials. As used herein, a composition is "substantially anhydrous" if it comprises less than about 10% water. Such particularly preferred delivery systems are known to those skilled in the art of absorbent articles. For example U.S. Patent 5,643,588, issued to Roe et al on July 1, 1997, describes diaper containing a liquid pervious topsheet coated with a lotion composition that reduces the adherence of feces to the skin of a wearer.

The delivery system may provide the proton donating active as a structural component of any of the structures included in the absorbent article. That is, such actives may be incorporated directly by known methods within the structure of the topsheet, the backsheet or one of the absorbent core materials during manufacture or assembly. For example, such actives may be incorporated into a polymeric structure such as a fiber as would be used to form the topsheet or the core or into a film as would be used to form the backsheet by means such as compounding or mixing during extrusion. Such a material could then "bloom" to the surface of the structure where it would be available for transfer to a wearer's skin.

In a particularly preferred embodiment of the invention the proton donating active is in particulate form and is dispersed throughout a hydrophobic skin care composition deliverable to the wearer's skin from at least a portion of a wearer-contacting surface, such as a top sheet, side panel, waist region, leg cuff, fastening tab, and the like of a disposable absorbent article. Suitable skin care compositions are discussed below. In an alternative embodiment, the proton donating active is incorporated as an aqueous solution into a substantially oleaginous skin care composition (e.g. a water-in-oil emulsion) transferable from at least a portion of a wearer-contacting surface to the wearer's skin. Suitable skin care compositions for delivering a proton donating active are described further below. In these preferred embodiments, the skin care composition may comprise between about 0.01% and about 40%, preferably between about 0.5% and about 20%, and more preferably between about 3% and about 7% by weight of the proton donating active. In these embodiments, at least a portion of the skin care composition is transferred from the article to the wearer's skin during normal usage of the article. Repeated application of such treated articles to the wearer's skin provides an available source from which the proton donating active transfers onto the skin continuously over time and accumulates to help maintain the wearer's skin at an acidic pH. Preferably, a minimum effective concentration of the proton donating active is delivered to the skin upon application of a single absorbent article.

Further, in many cases, that portion of the preferred skin care composition containing a proton donating active that does not transfer to a wearer's skin may still be effective in helping maintain the pH of the environment adjacent to the wearer's skin at an acidic pH. For example, such residual skin care composition would be effective in countering a pH increase due to any ammonia present in urine that comes in contact with a wearer-contacting surface of the absorbent article.

V. <u>Skin Care Compositions</u>

Skin care compositions suitable for use in the preferred embodiments of the invention are described in U.S. Patent Applications Serial Nos. 08/926,532 and 08/926,533, each filed on September 10, 1997; U.S. Patent No. 5,607,760, issued March 4, 1997; U.S. Patent No. 5,609,587, issued March 11, 1997; U.S. Patent No. 5,635,191, issued June 3, 1997; and U.S. Patent No. 5,643,588, issued July 1, 1997.

In addition to its function as a vehicle for delivering an effective concentration of a proton donating active to a wearers skin, the skin care composition that comprises the proton donating active may also comprise ingredients that, for example, reduce the adherence of feces to skin (e.g., to improve the ease of bowel movement clean up), provide a skin/feces barrier function (e.g., to coat the skin to prevent the adherence of feces) while remaining relatively liquid impervious but vapor pervious), or provide other therapeutic benefits to the skin (e.g., improve skin softness, maintain

or improve skin health), and the like. The skin care composition may be in a variety of forms, including, but not limited to, emulsions, lotions, creams, ointments, salves, suspensions, encapsulations, gels, and the like.

In order to deliver an effective concentration of the proton donating active to the skin via an absorbent article over time, an effective amount of the skin care composition applied to or migrated to one or more of the wearer-contacting surfaces of the article depends, to a large extent, on the particular composition used. The quantity of the composition on at least a portion of the wearer-contacting surface of the absorbent article preferably ranges from about 0.05 mg/in$^2$ (0.0078 mg/cm$^2$) to about 230 mg/in$^2$ (36 mg/cm$^2$), more preferably from about 1 mg/in$^2$ (0.16 mg/cm$^2$) to about 80 mg/in$^2$ (12 mg/cm$^2$), still more preferably from about 4 mg/in$^2$ (0.6 mg/cm$^2$) to about 52 mg/in$^2$ (8 mg/cm$^2$). However, these ranges are by way of illustration only and the skilled artisan will recognize that the nature of the composition will dictate the level that must be applied to deliver an effective concentration of the proton donating active and that the desirable level is ascertainable by routine experimentation in light of the present disclosure.

While the amount of skin care composition applied to the absorbent article is an important aspect of the present invention, more important is the amount of composition transferred to the wearer's skin during use of one or more treated articles. Though the amount of the proton donating active-containing composition delivered to the skin will depend to some degree on the nature of the composition employed, relatively low amounts may be delivered while still providing a minimum effective concentration of the proton donating active to the skin. This is particularly true for preferred compositions, such as that described in Example 1.

To determine the amount of proton donating active transferred to a wearer's skin after wearing one or more treated articles, a method is provided in the TEST METHODS section below for determining the amount of skin care composition transferred to the skin. Because the concentration of proton donating active in the skin care composition is known (i.e., a selected amount between about 0.01% and about 10% by weight), the amount of the proton donating active delivered to the skin may be extrapolated. With regard to the level of skin care composition that is transferred to the wearer during use of one treated absorbent article worn for a period of about 3 hours (a typical daytime wear time), particularly for preferred skin care compositions such as that described in Example 2, it is preferred that at least about 0.01 mg/in$^2$ (0.0016 mg/cm$^2$), more preferably at least about 0.05 mg/in$^2$ (0.0078 mg/cm$^2$), still more preferably at least about 0.1 mg/in$^2$ (0.016 mg/cm$^2$), of the composition is transferred to the skin over a three hour wear period. Typically, the amount of 2 composition delivered by one treated article will be from about 0.01 mg/in (0.0016 mg/cm$^2$) to about 5 mg/in (0.78 mg/cm$^2$), more preferably from about 0.05 mg/in$^2$ (0.0078 mg/cm$^2$) to about 3 mg/in$^2$ (0.47 mg/cm$^2$ ), still more preferably from about 0.1 mg/in$^2$ (0.016mg/cm$^2$)to about 2 mg/in$^2$ (0.31 mg/cm$^2$), over a three hour wear period.

For continual use of treated articles (in other words, changes occur in accordance with normal use patterns, which typically include changes every 3 to 4 hours during the day and a fresh article before overnight sleep) such as for a period of 24 hours, it will be preferred that at least about 0.03 mg/in$^2$ (0.0047 mg/cm$^2$), more preferably at least about 0.1 mg/in$^2$ (0.016 mg/cm$^2$), still more preferably at least about 0.3 mg/in$^2$ (0.047 mg/cm$^2$), of the composition is transferred to the wearer's skin over the 24 hour period. Typically, the amount of composition delivered after a period of 24 hours where treated articles are applied at each change, will be from about 0.03 mg/in$^2$ (0.0047 mg/cm$^2$) to about 18 mg/in$^2$ (2.79 mg/cm$^2$), more typically from about 0.1 mg/in$^2$ (0.016 mg/cm$^2$) to about 10 mg/in$^2$ (1.55mg/cm$^2$), still more typically from about 0.3 mg/in$^2$ (0.047 mg/cm$^2$) to about 6 mg/in$^2$ (0.93 mg/cm$^2$).

It will be recognized that of the numerous materials useful in the proton donating active-containing skin care compositions delivered to skin in accordance with the invention, those that have been deemed safe and effective skin care agents are logical materials for use herein. Such materials include Category I actives as defined by the U.S. Food and Drug Administration's (FDA) Tentative Final Monograph on Skin Protectant Drug Products for Over-the-Counter Human Use (21 C.F.R. § 347), which presently include: allantoin, aluminum hydroxide gel, calamine, cocoa butter, dimethicone, cod liver oil (in combination), glycerin, kaolin, petrolatum, lanolin, mineral oil, shark liver oil, white petrolatum, talc, topical starch, zinc acetate, zinc carbonate, zinc oxide, and the like. Other potentially useful materials are Category III actives as defined by the U.S. Food and Drug Administration's Tentative Final Monograph on Skin Protectant Drug Products for Over-the-Counter Human Use (21 C.F.R. § 347), which presently include: live yeast cell derivatives, aldioxa, aluminum acetate, microporous cellulose, cholecalciferol, colloidal oatmeal, cysteine hydrochloride, dexpanthenol, Peruvean balsam oil, protein hydrolysates, racemic methionine, sodium bicarbonate, Vitamin A, and the like.

Many of the FDA monographed skin care ingredients are currently utilized in commercially available skin care products, such as A and D® Ointment, Vaseline® Petroleum Jelly, Desitin® Diaper Rash Ointment and Daily Care® ointment, Gold Bond® Medicated Baby Powder, Aquaphor® Healing Ointment, Baby Magic® Baby Lotion, and Johnson's Ultra Sensitive® Baby Cream. An effective concentration of a proton donating active may be incorporated into any of these commercial products and applied to absorbent articles to create treated articles for use in the present invention.

As discussed further hereinafter, the skin care compositions useful for transferring proton donating actives to the skin of the wearer preferably, though not necessarily, have a melting profile such that they are relatively immobile and localized on the wearer-contacting surface of the article at room temperature, are readily transferable to the wearer at body temperature, and yet are not completely liquid under extreme storage conditions. Preferably, the compositions are easily transferable to the skin by way of normal contact, wearer motion, and/or body heat. Because the composition

preferably is substantially immobilized on the article's wearer-contacting surface, relatively low levels of composition are needed to impart the desired skin care benefits. In addition, special barrier or wrapping materials may be unnecessary in packaging the treated articles useful in the methods of the present invention.

In an alternative embodiment, the skin care compositions useful herein are water-in-oil emulsions, wherein the proton donating active is in the aqueous phase. However, the skin care composition itself may be solid (i.e., the aqueous phase is trapped within a solid oleaginous phase) or more often semi-solid, at 20°C, i.e. at ambient temperatures. By "semisolid" is meant that the composition has a rheology typical of pseudoplastic or plastic liquids. When no shear is applied, the compositions can have the appearance of a semi-solid but can be made to flow as the shear rate is increased. This is due to the fact that, while the composition contains primarily solid components, it also includes a liquid component. Preferably, the proton donating active-containing compositions of the present invention have a zero shear viscosity between about 1.0 X $10^6$ centipoise and about 1.0 X $10^8$. More preferably, the zero shear viscosity is between about 5.0 X $10^6$ centipoise and about 5.0 X $10^7$ centipoise. As used herein the term "zero shear viscosity" refers to a viscosity measured at very low shear rates (e.g., 1.0 $see^{-1}$) using plate and cone viscometer (a suitable instrument is available from TA Instruments of New Castle, DE as model number CSL 100). One of skill in the art will recognize means other than high melting point components (as are discussed below) can be used to provide zero shear viscosities. Exemplary means include establishing a structure having a yield value using components such as clays or fumed silica as is known in the art. Zero shear viscosity can also measured for such compositions comprising such alternative means by extrapolating a plot of viscosity vs. shear rate to a shear rate of zero at a temperature of about 20°C.

Preferred skin care compositions are at least semi-solid at room temperature to minimize composition migration. In addition, the compositions preferably have a final melting point (100% liquid) above potential "stressful" storage conditions that can be greater than 45°C (e.g., warehouse in Arizona, car trunk in Florida, etc.). Representative compositions having these melt characteristics are described in detail in U.S. Patent No. 5,643,588, U.S. Patent No. 5,607,760, U.S. Patent No. 5,609,587, and U.S. Patent No. 5,635,191.

Specifically, preferred compositions will have the following melt profile:

| Characteristic | Preferred Range | Most Preferred |
|---|---|---|
| % liquid at room temp. (20°C) | 2-50 | 3-25 |
| % liquid at body temp. (37°C) | 25-95 | 30-90 |
| final melting point (°C) | ≥38 | ≥45 |

By being solid or semisolid at ambient temperatures, preferred compositions containing the proton donating actives do not have a tendency to flow and migrate to a significant degree to undesired locations of the article to which they are applied. This means less skin care composition is required for imparting desirable therapeutic, protective and/or conditioning benefits.

To enhance immobility of preferred compositions, the viscosity of the formulated compositions should be as high as possible to prevent flow within the article to undesired location. Unfortunately, in some instances, higher viscosities may inhibit transfer of composition to the wearer's skin. Therefore, a balance should be achieved so the viscosities are high enough to keep the compositions localized on the surface of the article, but not so high as to impede transfer to the wearer's skin. Suitable viscosities for the compositions will typically range from about 5 to about 500 centipoise, preferably from about 5 to about 300 centipoise, more preferably from about 5 to about 100 centipoise, measured at 60°C using a rotational viscometer (a suitable viscometer is available from Lab Line Instruments, Inc. of Melrose Park, IL as Model 4537). The viscometer is operated at 60 rpm using a number 2 spindle.

For skin care compositions designed to provide a therapeutic and/or skin protective benefit in addition to the benefit derived from the proton donating active(s), a useful active ingredient in these compositions is one or more skin protectants or emollients. As used herein, the term an "emollient" is a material that protects against wetness or irritation, softens, soothes, supples, coats, lubricates, moisturizes, protects and/or cleanses the skin. (It will be recognized that several of the monographed actives listed above are "emollients", as that term is used herein.) In a preferred embodiment, such emollients will have a plastic or a liquid consistency at ambient temperatures, i.e., about 20-25°C.

Representative emollients useful in the present invention include, but are not limited to, emollients that are petroleum-based; sucrose esters of fatty acids; polyethylene glycol and derivatives thereof; humectants; fatty acid ester type; alkyl ethoxylate type; fatty acid ester ethoxylates; fatty alcohol type; polysiloxane type; propylene glycol and derivatives thereof; glycerin and derivatives thereof, including glyceride, acetoglycerides, and ethoxylated glycerides of $C_{12}$-$C_{28}$ fatty acids; triethylene glycol and derivatives thereof; spermaceti or other waxes; fatty acids or fatty alcohol ethers, particularly those having from 12 to 28 carbon atoms in their fatty chain, such as stearic acid and methyl stearyl ether; propoxylated fatty alcohols; other fatty esters of polyhydroxy alcohols; lanolin and its derivatives; kaolin and its derivatives; any of the monographed skin care agents listed above; or mixtures of these emollients. Suitable petroleum-based emollients include those hydrocarbons, or mixtures of hydrocarbons, having chain lengths of from 16 to 32 carbon atoms. Petroleum based hydrocarbons having these chain lengths include mineral oil (also known as "liquid petrolatum") and petrolatum (also known as "mineral wax," "petroleum jelly" and "mineral jelly"). Mineral oil usually refers to less viscous mixtures of. Petrolatum usually refers to more viscous mixtures of hydrocarbons. Petrolatum and mineral oil are particularly preferred emollients for compositions of the present invention.

Suitable fatty acid ester type emollients include those derived from $C_{12}$-$C_{28}$ fatty acids, preferably $C_{16}$-$C_{22}$ saturated fatty acids, and short chain ($C_1$-$C_8$, preferably $C_1$-$C_3$) monohydric alcohols. Representative examples of such esters include methyl palmitate, methyl stearate, isopropyl laurate, isopropyl myristate, isopropyl palmitate, ethylhexyl palmitate and mixtures thereof. Suitable fatty acid ester emollients can also be derived from esters of longer chain fatty alcohols ($C_{12}$-$C_{28}$, preferably $C_{12}$-$C_{16}$) and shorter chain fatty acids e.g., lactic acid, such as lauryl lactate and cetyl lactate.

Suitable fatty ester type emollients also include polyolpolyesters as described in U.S. Patent 5,609,587, issued to Roe on March 11, 1997, the disclosure of which is incorporated herein by reference. Exemplary polyols include, but are not limited to, polyhydric compounds such as pentaerythritol; sugars such as raffinose, maltodextrose, galactose, sucrose, glucose, xylose, fructose, maltose, lactose, mannose and erythrose; and sugar alcohols such as erythritol, xylitol, malitol, mannitol and sorbitol. Such polyols are esterified with fatty acids and/or other organic radicals having at least two carbon atoms and up to 30 carbon atoms. While it is not necessary that all of the hydroxyl groups of the polyol be esterified, preferred polyolpolyester emollients of the present invention have substantially all (e.g. at least about 85%) of the hydroxyl groups esterified. Particularly preferred are sucrose polyolpolyesters such as sucrose polycottonate, sucrose polysoyate, and sucrose polybehenate. Mixtures of such polyolpolyesters are also suitable emollients for the present invention.

Suitable alkyl ethoxylate type emollients include $C_{12}$-$C_{22}$ fatty alcohol ethoxylates having an average degree of ethoxylation of from about 2 to about 30. Preferably, the fatty alcohol ethoxylate emollient is selected from the group consisting of lauryl, cetyl, and stearyl ethoxylates, and mixtures thereof, having an average degree of ethoxylation ranging from about 2 to about 23. Representative examples of such alkyl ethoxylates include laureth-3 (a lauryl ethoxylate having an average degree of ethoxylation of 3), laureth-23 (a lauryl ethoxylate having an average degree of ethoxylation of 23), ceteth-10 (a cetyl alcohol ethoxylate having an average degree of ethoxylation of 10) and steareth-10 (a stearyl alcohol ethoxylate having an average degree of ethoxylation of 10). When employed, these alkyl ethoxylate emollients are typically used in combination with the petroleum-based emollients, such as petrolatum, at a weight ratio of alkyl ethoxylate emollient to petroleum-based emollient of from about 1:1 to about 1:5, preferably from about 1:2 to about 1:4.

Suitable fatty alcohol type emollients include $C_{12}$-$C_{22}$ fatty alcohols, preferably $C_{16}$-$C_{18}$ fatty alcohols. Representative examples include cetyl alcohol and stearyl alcohol, and mixtures thereof, such as cetearyl alcohol (Available from the Procter & Gamble Company, Cincinnati, OH as TA1618). When employed, these fatty alcohol emollients are typically used in combination with the petroleum-based emollients, such as petrolatum, at a weight ratio of fatty alcohol emollient to petroleum-based emollient of from about 1:1 to about 1:5, preferably from about 1:1 to about 1:2.

Other suitable types of emollients for use herein include polysiloxane compounds. In general, suitable polysiloxane materials for use in the present invention include those having monomeric siloxane units of the following structure:

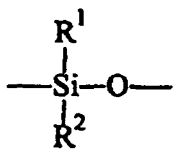

wherein, $R^1$ and $R^2$, for each independent siloxane monomeric unit can each independently be hydrogen or any alkyl, aryl, alkenyl, alkaryl, arakyl, cycloalkyl, halogenated hydrocarbon, or other radical. Any of such radicals can be substituted or unsubstituted. $R^1$ and $R^2$ radicals of any particular monomeric unit may differ from the corresponding functionalities of the next adjoining monomeric unit. Additionally, the polysiloxane can be either a straight chain, a branched chain or have a cyclic structure. The radicals $R^1$ and $R^2$ can additionally independently be other silaceous functionalities such as, but not limited to, siloxanes, polysiloxanes, silanes, and polysilanes. The radicals $R^1$ and $R^2$ may contain any of a variety of organic functionalities including, for example, alcohol, carboxylic acid, phenyl, and amine functionalities.

Exemplary alkyl radicals are methyl, ethyl, propyl, butyl, pentyl, hexyl, octyl, decyl, octadecyl, and the like. Exemplary alkenyl radicals are vinyl, allyl, and the like. Exemplary aryl radicals are phenyl, diphenyl, naphthyl, and the like. Exemplary alkaryl radicals are toyl, xylyl, ethylphenyl, and the like. Exemplary aralkyl radicals are benzyl, alphaphenylethyl, beta-phenylethyl, alpha-phenylbutyl, and the like. Exemplary cycloalkyl radicals are cyclobutyl, cyclopentyl, cyclohexyl, and the like. Exemplary halogenated hydrocarbon radicals are chloromethyl, bromoethyl, tetrafluorethyl, fluorethyl, trifluorethyl, trifluorotloyl, hexafluoroxylyl, and the like.

The viscosity of polysiloxanes useful in the present invention may vary as widely as the viscosity of polysiloxanes in general varies, so long as the polysiloxane is flowable or can be made to be flowable for application to the absorbent article. This includes, but is not limited to, viscosity as low as 5 centistokes (at 37°C as measured by a glass capillary viscometer according to ASTM standard method D-445) to about 20,000,000 centistokes. Preferably the polysiloxanes have a viscosity at 37°C ranging from about 5 to about 5,000 centistokes, more preferably from about 5 to about 2,000 centistokes, most preferably from about 100 to about 1000 centistokes. High viscosity polysiloxanes which themselves are resistant to flowing can be effectively deposited upon the absorbent articles by such methods as, for example, emulsifying the polysiloxane in surfactant or providing the polysiloxane in solution with the aid of a solvent, such as hexane, listed for exemplary purposes only. Particular methods for applying polysiloxane emollients to absorbent articles are discussed in more detail hereinafter.

Preferred polysiloxanes compounds for use in the present invention are disclosed in U.S. Patent 5,059,282, issued to Ampulski et al on October 22, 1991. Particularly preferred polysiloxane compounds for use as emollients in the compositions of the present invention include phenyl-functional polymethylsiloxane compounds (e.g., Dow Corning 556 Cosmetic-Grade Fluid: polyphenylmethylsiloxane) and cetyl or stearyl functionalized dimethicones such as Dow 2502 and Dow 2503 polysiloxane liquids, respectively. In addition to such substitution with phenyl-functional or alkyl groups, effective substitution may be made with amino, carboxyl, hydroxyl, ether, polyether, aldehyde, ketone, amide, ester, and thiol groups. Of these effective substituent groups, the family of groups comprising phenyl, alkyl, carboxyl, amino and hydroxyl groups are more preferred than the others; with amino and phenyl-functional groups being most preferred.

Suitable humectants include glycerin, propylene glycol, sorbitol, trihydroxy stearin, and the like.

When present, the amount of emollient that can be included in the composition will depend on a variety of factors, including the particular emollient involved, the skin benefits desired, the other components in the composition and like factors. The composition will typically comprise from about 10 to about 95% of the emollient. Preferably from about 20 to about 80%, and more preferably from about 40 to about 75%, by weight, of the emollient.

Another optional, preferred component of the proton donating active-containing skin compositions useful in the present invention is an agent capable of immobilizing the composition (including the proton donating active, the preferred emollient and/or other skin condition/protective agents) in the desired location in or on the treated article. Because certain of the preferred emollients in the composition have a plastic or liquid consistency at 20°C, they tend to flow or migrate, even when subjected to modest shear. When applied to a wearer-contacting surface or other location of an absorbent article, especially in a melted or molten state, the emollient will not remain primarily in or on the treated region. Instead, the emollient will tend to migrate and flow to undesired regions of the article.

Specifically, if the emollient migrates into the interior of the article, it can cause undesired effects on the absorbency of the article core due to the hydrophobic characteristics of many of the emollients and other skin conditioning agents used in the compositions useful in the present invention. It also means that much more emollient has to be applied to the article to get the desired therapeutic and/or protective benefits. Increasing the level of emollient not only increases the cost, but also exacerbates the undesirable effect on the absorbency of the article's core and undesired transfer of composition during processing/converting of the treated articles.

The immobilizing agent counteracts this tendency of the emollient to migrate or flow by keeping the emollient primarily localized on the surface or in the region of the article to which the composition is applied. This is believed to be due, in part, to the fact that the immobilizing agent raises the melting point and/or viscosity of the composition above that of the emollient. Since the immobilizing agent is preferably miscible with the emollient (or solubilized in the emollient with the aid of an appropriate emulsifier), it entraps the emollient on the surface of the article's body contacting surface or in the region to which it is applied.

It is also advantageous to "lock" the immobilizing agent on the body contacting surface or the region of the article to which it is applied. This can be accomplished by using immobilizing agents which quickly set up (i.e., solidify) upon application to the article. In addition, outside cooling of the treated article via blowers, fans, cold rolls, etc. can reduce the set up time of the immobilizing agent.

In addition to being miscible with (or solubilized in) the emollient, the immobilizing agent will, in one preferred embodiment, have a melting profile that will provide a composition that is solid or semisolid at ambient temperature. In this regard, preferred immobilizing agents will have a melting point of at least about 35°C. This is so the immobilizing agent itself will not have a tendency to migrate or flow. Preferred immobilizing agents will have melting points of at least about 40°C. Typically, the immobilizing agent will have a melting point in the range of from about 50° to about 150°C.

When utilized, immobilizing agents useful herein can be selected from any of a number of agents, so long as the acidifying properties of the skin care composition provide the skin benefits described herein. Preferred immobilizing agents will comprise a member selected from the group consisting of $C_{14}$-$C_{22}$ fatty alcohols, $C_{12}$-$C_{22}$ fatty acids, and $C_{12}$-$C_{22}$ fatty alcohol ethoxylates having an average degree of ethoxylation ranging from 2 to about 30, and mixtures thereof. Preferred immobilizing agents include $C_{16}$-$C_{18}$ fatty alcohols, most preferably crystalline high melting materials selected from the group consisting of cetyl alcohol, stearyl alcohol, behenyl alcohol, and mixtures thereof. (The linear

structure of these materials can speed up solidification on the treated absorbent article.) Mixtures of cetyl alcohol and stearyl alcohol, such as cetearyl alcohol (Available from the Procter & Gamble Company, Cincinnati, OH as TA1618), also are suitable. Other preferred immobilizing agents include $C_{16}$-$C_{18}$ fatty acids, most preferably selected from the group consisting of palmitic acid, stearic acid, and mixtures thereof. Mixtures of palmitic acid and stearic acid are particularly preferred. Still other preferred immobilizing agents include $C_{16}$-$C_{18}$ fatty alcohol ethoxylates having an average degree of ethoxylation ranging from about 5 to about 20. Preferably, the fatty alcohols, fatty acids and fatty alcohols are linear. Importantly, these preferred immobilizing agents such as the $C_{16}$ - $C_{18}$ fatty alcohols increase the rate of crystallization of the composition causing the composition to crystallize rapidly onto the surface of the substrate.

Other types of immobilizing agents that may be used herein include polyhydroxy fatty acid esters, polyhydroxy fatty acid amides, and mixtures thereof. Preferred esters and amides will have three or more free hydroxy groups on the polyhydroxy moiety and are typically nonionic in character. Because of the possible skin sensitivity of those using articles to which the composition is applied, these esters and amides should also be relatively mild and non-irritating to the skin.

Suitable polyhydroxy fatty acid esters for use in the present invention will have the formula:

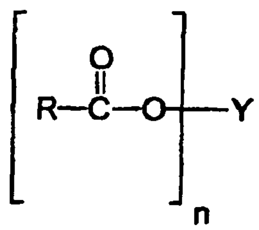

wherein R is a $C_5$-$C_{31}$ hydrocarbyl group, preferably straight chain $C_7$-$C_{19}$ alkyl or alkenyl, more preferably straight chain $C_9$-$C_{17}$ alkyl or alkenyl, most preferably straight chain $C_{11}$-$C_{17}$ alkyl or alkenyl, or mixture thereof; Y is a polyhydroxyhydrocarbyl moiety having a hydrocarbyl chain with at least 2 free hydroxyls directly connected to the chain; and n is at least 1. Suitable Y groups can be derived from polyols such as glycerol, pentaerythritol; sugars such as raffinose, maltodextrose, galactose, sucrose, glucose, xylose, fructose, maltose, lactose, mannose and erythrose; sugar alcohols such as erythritol, xylitol, malitol, mannitol and sorbitol; and anhydrides of sugar alcohols such as sorbitan.

One class of suitable polyhydroxy fatty acid esters for use in the present invention comprises certain sorbitan esters, preferably the sorbitan esters of $C_{16}$-$C_{22}$ saturated fatty acids. Because of the manner in which they are typically manufactured, these sorbitan esters usually comprise mixtures of mono-, di-, tri-, etc. esters. Representative examples of suitable sorbitan esters include sorbitan palmitates (e.g., SPAN 40), sorbitan stearates (e.g., SPAN 60), and sorbitan behenates, that comprise one or more of the mono-, di- and tri-ester versions of these sorbitan esters, e.g., sorbitan mono-, di- and tri-palmitate, sorbitan mono-, di- and tri-stearate, sorbitan mono-, di and tri-behenate, as well as mixed tallow fatty acid sorbitan mono-, di- and tri-esters. Mixtures of different sorbitan esters can also be used, such as sorbitan palmitates with sorbitan stearates. Particularly preferred sorbitan esters are the sorbitan stearates, typically as a mixture of mono-, di- and tri-esters (plus some tetraester) such as SPAN 60, available from ICI Surfactants of Wilmington, DE and sorbitan stearates sold under the trade name GLYCOMUL-S by Lonza, Inc. of Fair Lawn NJ. Although these sorbitan esters typically contain mixtures of mono-, di- and tri-esters, plus some tetraester, the mono- and di-esters are usually the predominant species in these mixtures.

Another class of suitable polyhydroxy fatty acid esters for use in the present invention comprises certain glyceryl monoesters, preferably glyceryl monoesters of $C_{16}$-$C_{22}$ saturated fatty acids such as glyceryl monostearate, glyceryl monopalmitate, and glyceryl monobehenate. Again, like the sorbitan esters, glyceryl monoester mixtures will typically contain some di- and triester. However, such mixtures should contain predominantly the glyceryl monoester species to be useful in the present invention.

Another class of suitable polyhydroxy fatty acid esters for use in the present invention comprise certain sucrose fatty acid esters, preferably the $C_{12}$-$C_{22}$ saturated fatty acid esters of sucrose. Sucrose monoesters and diesters are particularly preferred and include sucrose mono- and di-stearate and sucrose mono- and di- laurate.

Suitable polyhydroxy fatty acid amides for use in the present invention will have the formula:

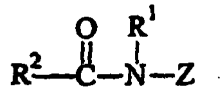

wherein $R^1$ is H, $C_1$-$C_4$ hydrocarbyl, 2-hydroxyethyl, 2-hydroxypropyl, methoxyethyl, methoxypropyl or a mixture thereof, preferably $C_1$-$C_4$ alkyl, methoxyethyl or methoxypropyl, more preferably $C_1$ or $C_2$ alkyl or methoxypropyl, most preferably $C_1$ alkyl (i.e., methyl) or methoxypropyl; and $R^2$ is a $C_5$-$C_{31}$ hydrocarbyl group, preferably straight chain $C_7$-$C_{19}$ alkyl or alkenyl, more preferably straight chain $C_9$-$C_{17}$ alkyl or alkenyl, most preferably straight chain $C_{11}$-$C_{17}$ alkyl or alkenyl, or mixture thereof; and Z is a polyhydroxyhydrocarbyl moiety having a linear hydrocarbyl chain with at least 3 hydroxyls directly connected to the chain. See U.S. patent 5,174, 927, issued to Honsa on December 29, 1992 which discloses these polyhydroxy fatty acid amides, as well as their preparation.

The Z moiety preferably will be derived from a reducing sugar in a reductive amination reaction; most preferably glycityl. Suitable reducing sugars include glucose, fructose, maltose, lactose, galactose, mannose, and xylose. High dextrose corn syrup, high fructose corn syrup, and high maltose corn syrup can be utilized, as well as the individual sugars listed above. These corn syrups can yield mixtures of sugar components for the Z moiety.

The Z moiety preferably will be selected from the group consisting of $-CH_2-(CHOH)_n-CH_2OH$, $-CH(CH_2OH)-[(CHOH)_{n-1}]-CH_2OH$, $-CH_2OH-CH_2-(CHOH)_2(CHOR^3)(CHOH)-CH_2OH$, where n is an integer from 3 to 5, and $R^3$ is H or a cyclic or aliphatic monosaccharide. Most preferred are the glycityls where n is 4, particularly $-CH_2-(CHOH)_4-CH_2OH$.

In the above formula, $R^1$ can be, for example, methyl, ethyl, propyl, isopropyl, butyl, 2-hydroxyethyl, methoxypropyl or 2-hydroxypropyl. $R^2$ can be selected to provide, for example, cocamides, stearamides, oleamides, lauramides, myristamides, capricamides, palmitamides, tallowamides, etc. The Z moiety can be 1-deoxyglucityl, 2-deoxyfructityl, 1-deoxymaltityl, 1-deoxylactityl, 1-deoxygalactityl, 1-deoxymannityl, 1-deoxymaltotriotityl, etc.

Certain of the polyolpolyester compounds that were discussed above as being suitable emollients are also suitable for use as immobilizing agents. Of particular utility is sucrose polybehenate. Such polyolpolyester immobilizing agents are discussed in detail in U.S. Patent 5,624,676, issued in the name of Mackey, et al. on April 29, 1997.

The most preferred polyhydroxy fatty acid amides have the general formula:

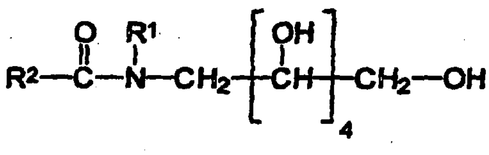

wherein $R^1$ is methyl or methoxypropyl; $R^2$ is a $C_{11}$-$C_{17}$ straight-chain alkyl or alkenyl group. These include N-lauryl-N-methyl glucamide, N-lauryl-N-methoxypropyl glucamide, N-cocoyl-N-methyl glucamide, N-cocoyl-N-methoxypropyl glucamide, N-palmityl-N-methoxypropyl glucamide, N-tallowyl-N-methyl glucamide, or N-tallowyl-N-methoxypropyl glucamide.

As previously noted, some of the immobilizing agents may require an emulsifier for solubilization in the emollient. This is particularly the case for certain of the glucamides such as the N-alkyl-N-methoxypropyl glucamides having HLB values of at least about 7. Suitable emulsifiers will typically include those having HLB values below about 7. In this regard, the sorbitan esters previously described, such as the sorbitan stearates, having HLB values of about 4.9 or less have been found useful in solubilizing these glucamide immobilizing agents in petrolatum. Other suitable emulsifiers include Steareth-2 (polyethylene glycol ethers of stearyl alcohol that conform to the formula $CH_3(CH_2)_{17}(OCH_2CH_2)_nOH$, where n has an average value of 2), sorbitan tristearate, isosorbide laurate, and glyceryl monostearate. The emulsifier can be included in an amount sufficient to solubilize the immobilizing agent in the emollient such that a substantially homogeneous mixture is obtained. For example, an approximately 1:1 mixture of N-cocoyl-N-methyl glucamide and petrolatum that will normally not melt into a single phase mixture, will melt into a single phase mixture upon the addition of 20% of a 1:1 mixture of Steareth-2 and sorbitan tristearate as the emulsifier.

Other types of ingredients that can be used as immobilizing agents, either alone, or in combination with the above-mentioned immobilizing agents, include waxes such as carnauba, ozokerite, beeswax, candelilla, paraffin, ceresin, esparto, ouricuri, rezowax, isoparaffin, and other known mined and mineral waxes. The high melt point of these materials can help immobilize the composition on the desired surface or location on the article. Additionally microcryatalline waxes are effective immobilizing agents. Microcrystalline waxes can aid in "locking" up low molecular weight hydrocarbons within the skin care composition. Preferably the wax is a paraffin wax. An example of a particularly preferred alternate immobilizing agent is a paraffin wax such as Parrafin S.P. 434 from Strahl and Pitsch Inc. of West Babylon, NY.

The amount of the optional immobilizing agent that can be included in the composition will depend on a variety of factors, including the actives (e.g., emollients, proton donating actives, etc.) involved, the particular immobilizing agent involved, if any, the other components in the composition, whether an emulsifier is required to solubilize the immobilizing agent in the other components, and like factors. When present, the composition will typically comprise from about 5 to about 90% of the immobilizing agent. Preferably, the composition will comprise from about 5 to about 50%, most preferably

from about 10 to about 40%, of the immobilizing agent.

It is highly desirable that at least a portion of the article's topsheet be made of a hydrophilic material to promote rapid transfer of liquids (e.g., urine) through the topsheet. Similarly, it may be desirable that the composition be sufficiently wettable to ensure that liquids will transfer through the topsheet rapidly. Alternatively, hydrophobic skin care compositions may be utilized, so long as they are applied such that the fluid handling properties of the topsheet are adequately maintained. (For example, as discussed below, nonuniform application of the composition to the topsheet is one means to accomplish this goal.) This diminishes the likelihood that body exudates will flow off the composition-treated topsheet rather than being drawn through the topsheet and being absorbed by the absorbent core. Where a hydrophilic composition is desired, depending upon the particular components used in the composition, a hydrophilic surfactant (or a mixture of hydrophilic surfactants) may, or may not, be required to improve wettability. For example, some immobilizing agents, such as N-cocoyl-N-methoxypropyl glucamide have HLB values of at least about 7 and are sufficiently wettable without the addition of hydrophilic surfactant. Other immobilizing agents such as the $C_{16}$ - $C_{18}$ fatty alcohols having HLB values below about 7 may require addition of hydrophilic surfactant to improve wettability when the composition is applied to article topsheets. Similarly, a hydrophobic emollient such as petrolatum may require the addition of a hydrophilic surfactant if a hydrophilic composition is desired. Of course, the concern around wettability is not a factor when the wearer-contacting surface under consideration is other than the article's topsheet or when fluid handling properties of the topsheet are adequately maintained via other means (e.g., nonuniform application).

Suitable hydrophilic surfactants will preferably be miscible with the other components of the skin care composition so as to form blended mixtures. Because of possible skin sensitivity of those using disposable absorbent products to which the composition is applied, these surfactants should also be relatively mild and non-irritating to the skin. Typically, these hydrophilic surfactants are nonionic to be not only non-irritating to the skin, but also to avoid other undesirable effects on any other structures within the treated article. For example, reductions in tissue laminate tensile strength, adhesive bond sufficiencies, and the like.

Suitable nonionic surfactants may be substantially nonmigratory after the composition is applied to the articles and will typically have HLB values in the range of from about 4 to about 20, preferably from about 7 to about 20. To be nonmigratory, these nonionic surfactants will typically have melt temperatures greater than the temperatures commonly encountered during storage, shipping, merchandising, and use of disposable absorbent products, e.g., at least about 30°C. In this regard, these nonionic surfactants will preferably have melting points similar to those of the immobilizing agents previously described.

Suitable nonionic surfactants for use in compositions that will be applied to the articles, at least in the liquid discharge region of the diaper, include alkylglycosides; alkylglycoside ethers as described in U.S. Patent 4,011,389, issued to Langdon, et al on March 8, 1977; alkylpolyethoxylated esters such as Pegosperse 1000MS (available from Lonza, Inc., Fair Lawn, NJ), ethoxylated sorbitan mono-, di- and/or tri-esters of $C_{12}$-$C_{18}$ fatty acids having an average degree of ethoxylation of from about 2 to about 20, preferably from about 2 to about 10, such as TWEEN 60 (sorbitan esters of stearic acid having an average degree of ethoxylation of about 20) and TWEEN 61 (sorbitan esters of stearic acid having an average degree of ethoxylation of about 4), and the condensation products of aliphatic alcohols with from about 1 to about 54 moles of ethylene oxide. The alkyl chain of the aliphatic alcohol is typically in a straight chain (linear) configuration and contains from about 8 to about 22 carbon atoms. Particularly preferred are the condensation products of alcohols having an alkyl group containing from about 8 to about 22 carbon atoms with from about 2 to about 30 moles of ethylene oxide per mole of alcohol (on average). Examples of such ethoxylated alcohols include the condensation products of myristyl alcohol with 7 moles of ethylene oxide per mole of alcohol, the condensation products of coconut alcohol (a mixture of fatty alcohols having alkyl chains varying in length from 10 to 14 carbon atoms) with about 6 moles of ethylene oxide. A number of suitable ethoxylated alcohols are commercially available, including TERGITOL 15-S-9 (the condensation product of $C_{11}$-$C_{15}$ linear alcohols with 9 moles of ethylene oxide), marketed by Union Carbide Corporation of Danbury, CT; the NEODOL brand name surfactants marketed by Shell Chemical Co. of Houston, TX, in particular NEODOL 25-12 (condensation product of $C_{12}$-$C_{15}$ linear alcohols with an average of 12 moles of ethylene oxide) and NEODOL 23-6.5T (condensation product of $C_{12}$-$C_{13}$ linear alcohols with an average of 6.5 moles of ethylene oxide that has been distilled (topped) to remove certain impurities), and especially the PLURAFAC brand name surfactants marketed by BASF Corp. of Mt. Olive, NJ, in particular PLURAFAC A-38 (a condensation product of a $C_{18}$ straight chain alcohol with 27 moles of ethylene oxide). (Certain of the hydrophilic surfactants, in particular ethoxylated alcohols such as NEODOL 25-12, can also function as alkyl ethoxylate emollients). Other examples of preferred ethoxylated alcohol surfactants include ICI's class of Brij surfactants and mixtures thereof, with Brij 72 (i.e., Steareth-2) and Brij 76 (i.e., Steareth-10) being especially preferred. Also, mixtures of cetyl alcohol and stearyl alcohol ethoxylated to an average degree of ethoxylation of from about 10 to about 20 may also be used as the hydrophilic surfactant.

Another type of suitable surfactant for use in the composition includes Aerosol OT, a dioctyl ester of sodium sulfosuccinic acid marketed by Cytec Industries, Inc. of Morristown, NJ.

Still another type of suitable surfactant for use in the composition includes silicone copolymers such as General Electric SF 1188 (a copolymer of a polydimethylsiloxane and a polyoxyalkylene ether) and General Electric SF 1228 (a

silicone polyether copolymer). These silicone surfactants can be used in combination with the other types of hydrophilic surfactants discussed above, such as the ethoxylated alcohols. These silicone surfactants have been found to be effective at concentrations as low as 0.1 %, more preferably from about 0.25 to about 1.0%, by weight of the composition.

Where a hydrophilic composition is desired, the amount of hydrophilic surfactant required to increase the wettability of the composition to a desired level will depend in part upon the HLB value and level of immobilizing agent, if any, used, the HLB value of the surfactant used and like factors. The composition can comprise from about 0.1 to about 50% of the hydrophilic surfactant when needed to increase the wettability properties of the composition. Preferably, the composition comprises from about 1 to about 25%, most preferably from about 10 to about 20%, of the hydrophilic surfactant when needed to increase wettability.

Compositions can comprise other components typically present in emulsions, creams, ointment, lotions, powders, suspensions, etc. of this type. These components include water, viscosity modifiers, perfumes, disinfectant antibacterial actives, antiviral agents, vitamins, pharmaceutical actives, film formers, deodorants, opacifiers, astringents, solvents, preservatives, and the like. In addition, stabilizers can be added to enhance the shelf life of the composition such as cellulose derivatives, proteins and lecithin. All of these materials are well known in the art as additives for such formulations and can be employed in appropriate amounts in the compositions for use herein.

If water-based skin care compositions are used, a preservative will be needed. Suitable preservatives include propyl paraben, methyl paraben, benzyl alcohol, benzylkonnium chloride, tribasic calcium phosphate, BHT, or acids such as citric, tartaric, maleic, lactic, malic, benzoic, salicylic, and the like. Suitable viscosity increasing agents include some of the agents described as effective immobilizing agents. Other suitable viscosity increasing agents include alkyl galactomannan, silica, talc, magnesium silicate, sorbitol, colloidal silicone dioxide, magnesium aluminum silicate, zinc stearate, wool wax alcohol, sorbitan sesquioleate, cetyl hydroxyethyl cellulose and other modified celluloses. Suitable solvents include propylene glycol, glycerin, cyclomethicone, polyethylene glycols, hexylene glycol, diol and multi-hydroxy based solvents. Suitable vitamins include A, D-3, E, B-5 and E acetate.

## V1. Treating Articles With the Skin Care Composition

In preparing absorbent articles to carry out the methods of the present invention, the skin care composition containing the proton donating active is applied such that during wear, at least some portion of the composition will transfer from the treated article to the wearer's skin. That is, the skin care composition is either applied directly to one or more body contacting surfaces, or is applied in alternate locations or means such that the skin care composition is readily available for transfer from one or more body contacting surfaces during use without intervention by the user/caregiver. (For example, materials positioned beneath the body contacting surface, encapsulated compositions, etc.) Of course, to effectuate delivery of the composition to those body regions most susceptible to skin roughness, it will be preferred to include the composition on the portion of the topsheet and cuffs that will contact the wearer's buttocks, genitals, intertriginous and anal regions during wear. Additionally, the composition may be applied to other article regions for delivery to one or more of the wearer's hips, abdomen, back, waist, sides, thighs, etc. Suitable methods include spraying, printing (e.g., flexographic printing), coating (e.g., contact slot coating, gravure coating), extrusion, or combinations of these application techniques, e.g. spraying the skin care composition on a rotating surface, such as a roll surface, that then transfers the composition to the desired portion of the article. The skin care composition containing the proton donating active can also be applied as a solid material via any of a variety methods, for example extrusion.

When applied to the article's topsheet, the manner of applying the composition to the article should be such that the topsheet does not become saturated with the composition, at least in the region corresponding to the liquid discharge region of the article, if the composition is hydrophobic in nature. If the topsheet becomes saturated with the composition in the liquid discharge region, there is a greater potential for the composition to block the topsheet openings, reducing the ability of the topsheet to transmit liquid to the underlying absorbent core. Also, saturation of the topsheet is not required to obtain the therapeutic and/or protective benefits. Similarly, saturation of other treated article components may not be necessary or desired to transfer sufficient composition for desired skin benefits. Particularly suitable application methods will apply the composition primarily to the outer surface of the topsheet of the article.

The minimum level of the composition containing the proton donating active to be applied to the article's wearer-contacting surface is an amount effective for providing the therapeutic, protective and/or skin conditioning benefits when the composition is delivered pursuant to the present invention. The level of composition applied will depend on various factors, including the article component treated, the relative amount of surface area of the wearer-contacting surface not treated with the composition, the composition's content and the like. In general, with compositions that are relatively hydrophobic and are to be applied to essentially all of the topsheet, the composition is preferably applied to the article topsheet in an amount ranging from about 0.1 mg/in$^2$ (0.016 mg/cm$^2$) to about 15 mg/in$^2$ (2.33 mg/cm$^2$), more preferably from about 1 mg/in$^2$ (0.16 mg/cm$^2$) to about 10 mg/in$^2$ (1.55 mg/cm$^2$). It will be recognized that higher levels of skin care composition may be applied to other article components where fluid handling properties are not impacted (e.g., cuffs, waist band, side panels, etc.). It will also be recognized that for compositions that are relatively hydrophilic, higher add-on levels may be used on the topsheet without adversely impacting liquid handling properties to an unacceptable degree. Conversely, higher levels of a hydrophilic composition may be undesired when applied to components (e.g., cuff, waist) other than the topsheet, to avoid wicking of exudates to the edges of the article which may result in leakage.

Because the composition is preferably substantially immobilized on the surface of the region treated, relatively small amounts of composition are needed to deliver an effective amount of the proton donating active. It is believed that the ability to use low levels to impart the desired skin benefits is due to the fact that the composition is continuously, automatically delivered as articles are worn. As indicated, the ability to use relatively low levels of skin care composition, allows the article's topsheet to maintain its liquid transfer properties in the liquid discharge region.

The composition can be applied nonuniformly to the body contacting surface of the article. By "nonuniform" it is meant that the amount, location, pattern of distribution, etc. of the composition can vary over the wearer-contacting surface, and may further vary over specific regions of the article. For example, to maintain the liquid handling performance of the topsheet, it may be desired to apply the composition nonuniformly to the topsheet, particularly if the composition is hydrophobic in nature. In this regard, some portions of the treated surface of the article (and regions thereof) can have greater or lesser amounts of composition, including portions of the surface that do not have any composition on it. When the composition is relatively hydrophobic, in one such preferred embodiment the surface of the topsheet will have regions where no composition is applied, particularly in areas of the topsheet that correspond to the crotch region of the article. As used herein, the crotch region of the article is the rectangle, defined below, that is centered longitudinally and laterally about the article's crotch point. The "crotch point" is determined by placing the article on a wearer in a standing position and then placing an extensible filament around the legs in a figure eight configuration. The point in the article corresponding to the point of intersection of the filament is deemed to be the crotch point of the article. (It is understood that the crotch point is determined by placing the absorbent article on a wearer in the intended manner and determining where the crossed filament would contact the article.) With regard to incontinence devices (e.g., diapers, adult incontinent articles), the length of the crotch region corresponds to 40% of the absorbent article's total length (i.e., in the y-dimension). With regard to sanitary napkins, the length of the crotch region corresponds to 80% of the absorbent article's total length. The width of the crotch region is equivalent to the width of the widest absorbent core component as measured at the crotch point. (As used herein, "absorbent core" components are those materials involved with acquiring, transporting, distributing and/or storing body liquids. As such, the term absorbent core does not include the topsheet or backsheet of the absorbent article.) By way of illustration, for an incontinent article having a length of 20 in. and a core width at the crotch point of 4 in., the crotch region is the rectangle, centered on the crotch point, having a length of 8 in. and a width of 4 in.

Surprisingly, while the topsheet or other components comprising the composition are treated nonuniformly (e.g., microscopic or macroscopic regions where no composition is applied), during wear of the article, the composition is transferred to the wearer even in regions of the skin corresponding to untreated regions within the topsheet or other components. The amount and uniformity of composition transferred to the skin is believed to depend on several factors, including, for example, application pattern of the skin care composition, contact of the wearer's skin to the treated article surface, friction created during wear time between the wearer's skin and the treated region, warmth generated from wearer to enhance the transfer of the composition, the composition's properties, the materials which constitute the composition, and the like.

Where the composition is applied nonuniformly, any pattern may be utilized, including, for example, application of small droplets (obtained via, e.g., spraying) discrete dots (obtained via, e.g., gravure printing), stripes that run in the longitudinal or lateral direction of the article (obtained via contact slot coating), spirals that run in the longitudinal or lateral direction, etc., patterned prints, etc. In those embodiments where the topsheet comprises discrete, untreated regions, the percent open area of the region of the topsheet that corresponds to the crotch region of the article can vary widely. (As referred to herein, the "percent open area" of the topsheet is determined by (i) measuring the surface area of the topsheet that overlies the crotch region, (ii) measuring the total surface area of the untreated region(s) in this portion of the topsheet and (iii) dividing the measurement in (ii) by the measurement in (i). As used herein, "untreated" means a region of the topsheet having less than about 0.01 mg/in$^2$ (0.0016 mg/cm$^2$) of the composition. In this regard, the percent open area may be from about 1% to about 99%, from about 5% to about 95%, from about 10% to about 90%, from about 15% to about 85%, from about 20% to about 80%, from about 25% to about 75%, from about 30% to about 70%, or from about 35% to about 65%. The percent open area required to achieve the desired composition effect and the desired liquid handling properties of the topsheet will be dictated largely by the characteristics of the composition (in particular the composition's contents and its relative hydrophobicity/hydrophilicity properties). One skilled in the art will appreciate that the desired percent open area will be readily determined through routine experimentation.

In general, with compositions that are relatively hydrophobic and are to be applied such that regions of the topsheet are not coated with the composition, the composition is preferably applied to the article topsheet in an amount ranging from about 0.05 mg/in$^2$ (0.0078 mg/cm$^2$) to about 35 mg/in$^2$ (5.43 mg/cm$^2$), more preferably from about 1mg/in$^2$ (0.16 mg/cm$^2$) to about 25 mg/in$^2$ (3.88 mg/cm$^2$), still more preferably 4 mg/in$^2$ (0.62 mg/cm$^2$) to about 20 mg/in$^2$ (3.1 mg/cm$^2$). It will be recognized that for compositions that are relatively hydrophilic, higher add-on levels may be used without adversely impacting liquid handling properties of the topsheet to an unacceptable degree. Of course, for articles

having relatively high percent open areas in the crotch, greater add-on levels may be obtainable without adversely affecting liquid handling by the topsheet.

In one preferred embodiment for carrying out the present methods, the topsheet of the articles utilized will comprise stripes of composition that run in the article's longitudinal direction. These longitudinal stripes (or spirals) are separated by longitudinal stripes where little or no composition is applied to the topsheet. In these embodiments, each stripe of composition will typically have a width of from about 0.1 in. to about 0.75 in., more typically from about 0.1 in. to about 0.5 in., and the width of the stripes containing no composition will typically be from about 0.1 in. to about 1 in., more typically from about 0.15 to about 0.5 in. These ranges are applicable to typical infant diaper designs. For larger products such as adult incontinent products, these ranges may be higher.

The skin care composition can also be applied in nonuniform patterns on other article components. In these cases, the open area is calculated by the rectangle defined by the perimeters of the skin care composition.

The composition can be applied to the article at any point during assembly. For example, the composition can be applied to the finished disposable absorbent product before it has been packaged. The composition can also be applied to a given component (e.g., topsheet, cuffs, sides, waist, etc.), at the converting site or by the material supplier, before it is combined with the other raw materials to form a finished disposable absorbent product. Again, the composition can be applied to other zones of the article such that the composition will migrate to one or more body contacting surfaces during use.

The composition is typically applied from a melt thereof to the article. Since in a preferred embodiment, the composition melts at a temperature significantly above ambient temperatures, it is usually applied as a heated composition to the article. Typically, the composition is heated to a temperature in the range from about 35° to about 150°C, preferably from 40° to about 100°C, prior to being applied to the article. The proton donating active may be added to the composition prior to or after heating. If added prior to heating, the temperature to which the composition is heated is selected so as not to inactivate the proton donating active. Alternatively, the proton donating active may be added to the pre-heated composition when it has cooled to a temperature that does not affect the proton donating active but is still sufficiently liquid to be applied to the article. Once the melted composition has been applied to the article, it is allowed to cool and solidify. Preferably, the application process is designed to aid in the cooling/set up of the composition.

In applying compositions to the articles, methods such as contact slot coating, spraying, gravure coating, extrusion coating methods are preferred. One such method involves slot coating of the composition on the article's topsheet after the topsheet is assembled with the other raw materials into a finished product.

## VII. Test Methods

### A. Skin pH Reduction Test

#### Test Material Preparation

1. Prepare a carrier lotion comprising 58 parts petrolatum, 41 parts stearyl alcohol, and one part aloe extract according to the method described in Example 2 below.

2. Determine the weight of the potential proton donating active required to provide 0.039 moles of hydrogen ion. For example, 2.5 grams of citric acid (MW = 192, 3 moles of hydrogen ion per mole of citric acid) provides 0.039 moles of hydrogen ion.

3. To the weight of material determined in step 2 add an amount of the carrier lotion prepared in step 1 to provide a total of 50 grams of test material. Mix well to distribute the potential proton donating active evenly throughout the carrier lotion. The test material is now ready for evaluation according to the method described below.

#### Apparatus

pH Meter — A suitable skin pH meter is available from Courage + Khazaka Electronic GmbH of Cologne, Germany as model number PH-900.

Electrolyte 3 molar potassium chloride

Procedure

Skin pH Meter Operation

1. Set the apparatus up and calibrate it according to the manufacturer's instructions.

2. Before starting a skin pH measurement, wash the probe with distilled water

3. Shake off the water to have not a wet, but a moist probe. Too much water at the membrane may influence the measuring result or delay a stable, informative display. Take care that the probe is not dry.

4. The probe head should always be in a position downwards and vertical with slight pressure onto the measuring skin area.

5. Start the measurement with the ON key. A clock appears on the display counting backwards for 3 seconds from 3 to 0.

6. A sound will follow after 3 seconds and the measurement value appears on the display. The instrument will shut off after 2 minutes when not in use. It is possible to start a new measurement by pressing the ON key at any time during these 2 minutes.

7. Shut down is signaled by a 5 times sound. To restart the instrument, press the ON key.

Skin pH Reduction Determination

1. Mark a rectangular area approximately 20 square centimeters on the subject's volar forearm for a carrier lotion control and for each potential proton donating active to be tested. Up to six areas can be marked per subject

2. Measure and record the background skin pH following the steps described above.

3. Apply about 20 milligrams per square centimeter of the carrier lotion that contains the potential proton donating active. The preparation method for this lotion/active composition is described above. After two minutes wipe off any excess lotion.

4. Repeat the measurement of skin pH after a further two minute equilibration time. Record the post application skin pH.

5. Repeat steps 1 through 4 for four additional subjects.

Data Reporting and Acceptance Criteria

1. Record the background skin pH and the post application skin pH for each of the four subjects.

2. Calculate the difference between background skin pH and post application skin pH ($\Delta$pH = (Background Skin pH) - (Post Application Skin pH))for each subject.

3. Calculate and report the average $\Delta$pH for each potential proton donating active.

4. A potential proton donating active is chemically suitable for the present invention if the average $\Delta$pH is positive (i.e. the post application skin pH is less than the background skin pH) at a confidence level of at least 90%

pH Reduction Duration

If there is a need or desire to determine the duration of a pH reduction when a potential buffer active ingredient is evaluated as described above, additional skin pH measurements can be made at any duration or durations after application of the potential proton donating active. When such testing is conducted the elapsed time since application and skin pH should be recorded for each measurement made.

B. Transfer of Skin Care Composition to Wearer's Skin

Overview

This method uses a removable skin analog material that is placed on a wearer's skin for a controlled period of time. After the skin analog has been removed, it is extracted using an appropriate solvent and the amount of skin care composition and, by extrapolation the amount of proton donating active, deposited thereon is determined using known analytical methods. The method is described for use with infant diapers comprising skin care compositions that either contain or do not contain proton donating active(s), as defined herein. One of skill in the art will recognize the appropriate changes for other skin care compositions, absorbent articles, or wearer types.

Subjects

Approximately equal numbers of male and female infants should be selected using the following inclusion and exclusion criteria. Sufficient infants should be selected to ensure that there are at least fifteen subjects per condition and transfer time who complete all aspects of the test.

Inclusion Criteria

a. Healthy infant

b. Caregiver willing to not use lotions, creams, powders or other skin preparations in the diaper area for the duration of the test.

c. Infants who wear disposable diapers full time

d. Caregiver willing to give child bath the evening before the study and not again until after completion of the study

e. Caregiver willing to have child refrain from swimming from the evening before the study until after completion of the study.

Exclusion Criteria

a. The infant has been ill within the last four days

b. Diarrhea (soft stool) any time during the four days before the test

c. Medication which might increase frequency of bowel movements (e.g., oral antibiotics, anti fungal agents, corticosteroids)

d. Damaged skin in or around the test site (e. g., from sunburn, active dermal lesions, or the like)

e. Known allergies or irritation from adhesive or skin care ingredients

Materials

| In Vivo Transfer | |
|---|---|
| Skin Analog: | Dermatological Tape-TEGADERM Tape No. 1622W available from 3M Health Care, St. Paul, MN |
| Sample Container | Glass jar with closure available from VWR Scientific, West Chester, PA as catalog Number 15900-242 |
| Tape Release Powder | Baby powder (comprising only talc and fragrance) available from Johnson & Johnson, New Brunswick, NJ |
| Surgical Gloves | Available from Best Manufacturing Co., Menlo GA, as product 6005PFM. |

| Extraction and Analysis | |
|---|---|
| Extraction Solvent | Dichloromethane, available from Sigma-Aldrich of St. Louis, MO as 27056-3 |
| Stearyl alcohol | Aldrich 25876-8 |
| 1-Hexadecanol | Aldrich 25874-1 |
| Dispensing Flask | 10 ml |
| Gas Chromatograph | Flame ionization Detector, Hewlett Packard Model 5890 is suitable. |
| Column | Capillary column: Chrompack CP Sil-5 CB, 2 meters X 0.25 mm id, 0.12 micron film thickness fused silica capillary (no substitutions) |
| Instrumental Data | Must be able to reproducibly determine areas of peaks of |
| System | interest. |

Method

In Vivo Transfer

A. Confirm from the subject's caregiver that the subject has been bathed within the last 24 hours and that no lotions, powders, etc. have been applied to the diapered region of the subject's skin since bathing.

B. Wearing the surgical gloves, place the subject on the table and remove his/her diaper.

C. Turn the subject on his/her stomach.

D. Remove the release liner from a TEGADERM tape and lightly brush J&J Baby Powder over the adhesive surface (Wear surgical gloves, or the like, during application to prevent contamination of the tape). Provide sufficient powder such that there is a light coat of powder over all of the tape except the edges. (This step is done to keep the tape from adhering too aggressively to the child's skin.).

E. Figures 2a and 2b illustrate placement location for the TEGADERM tape, shown in those figures as tape 700. Apply the tape 700 to the child's right buttock. The tape 700 is to be applied to the highest point on the child's buttock immediately adjacent to, but not in, the child's gluteal groove. A second tape 700 may be applied to measure transfer at two time increments or the effect of an additional diaper. If a second tape is used, apply the tape 700 on the left buttock using the procedure described above.

F. Change diapers according to the following protocol: 3 hour transfer time-1 diaper; 6 hour transfer time-2 diapers (change at 3 hours); 24 hour transfer times *ad lib* by caregiver. For 24 hour transfer times the following additional instructions are to be followed:

1. Use only water and a washcloth for cleaning the diapered area for the duration of the test. Do not use baby wipes. Avoid touching the area around the tapes with hands or any cleaning implement.

2. Do not use skin care products (lotions, ointments, creams, soap, etc.) for the duration of the test.

3. Do not bathe the subject for the duration of the test.

4. Use only the test diapers. Record the time of each diaper change.

5. Record the time of any bowel movement and clean the subject with water and a wash cloth.

G. Record the time each diaper was applied for all test diapers.

H. Recall the subject near the end of the predetermined transfer time.

I. Remove the test diaper. If the child has had a bowel movement, the study personnel should remove the tape 700 and discard it (the subject has then completed the test and data from that subject are not included in the analysis). If the subject has urinated, the tape 700 will be acceptable for analysis as described below.

J. Test facility personnel should wear surgical gloves and remove the tape 700 by grasping the edge of the tape 700 with tweezers and gently peeling the remaining portion of the tape 700 from the skin.

K. Place the used tape 700 in one of the glass jars and close the lid. Make sure the jar is properly labeled for subsequent sample identification.

L. At the completion of the test collect all of the samples in the jars for analysis as described below.

Extraction and Analysis

This method is designed for use with the preferred skin care composition, the skin care composition of Table 2. One of ordinary skill in the art will recognize what adaptations may be necessary to extract and analyze the level of other skin care compositions. In principle: 1) one of the major ingredients of the composition is extracted from the skin analog using an appropriate solvent; 2) gas chromatographic or other appropriate quantitative analytical techniques are then used to determine the level of the major ingredient in the extract; 3) amount of skin care composition is calculated per unit area based on amount of major ingredient in extract and the area of the tape.

Internal Standard/Extraction Solvent

Prepare an internal standard/extraction solvent by accurately weighing 100±2 mg of 1-hexadecanol into a small beaker. Dissolve the 1-hexadecanol in dichloromethane and transfer to a 1 liter volumetric flask. Rinse the beaker 3 more times with dichloromethane transferring each rinse portion to the volumetric flask. Fill the volumetric flask to volume and mix well. This solution will be used to deliver the internal standard and extract skin care composition from the tapes. When not being used, this container should be kept tightly capped to prevent evaporation of solvent.

Calibration Standard

Prepare a calibration standard of known concentration by accurately weighing (±0.1 mg) 10 ±1 mg of the stearyl alcohol into a 100 ml volumetric flask. Record the weight of stearyl alcohol used. Add the internal standard/extraction solvent to the flask and mix to dissolve. Fill to volume and mix well. When not being used, this container should be kept tightly capped to prevent evaporation of solvent. This solution will be used to determine the relative response of the stearyl alcohol to the 1-hexadecanol internal standard for calibration of the instrument.

Preparation and Calibration of the Gas Chromatograph

All equipment should be installed, operated and maintained according to manufacturer's recommendations.

Install the column and check all the gas flows with the column oven at 100°C and the injection port and detector at operating temperatures. The GC will be operated under the following conditions:

| | |
|---|---|
| Carrier Gas | Hydrogen (Helium may be used); flow rate 1.5 ml/min |
| Injection Port | 325°C; Split vent flow 30 ml/min; Septum purge 2 ml/min; straight through liner with glass wool plug; Merlin microseal. |
| Injection volume | 2 μl split |
| FID Detector | 350°C; set gas flows according to manufacturer suggestions. Typical gas flows are 400 ml/minute for air, 30 ml/minute for hydrogen and 30 ml/minute for the auxiliary (make up) gas. |
| Column Oven | 100°C ramped at 15°C / minute to 325 °C; hold for 10 minutes |

Insure that all connections are tight and leak free. Ignite the detector and allow it to stabilize. Condition the column at 325 °C for 30 minutes. Clean the syringe with dichloromethane as needed. The syringe should also be rinsed with dichloromethane several times after each injection. Make several blank runs with injections of dichloromethane to ensure that a good baseline is obtained and that no extraneous peaks are present in the chromatogram. If extraneous peaks are present or baseline is not suitable, trouble shoot and correct problem(s).

Calibrate the instrument using the calibration standard prepared previously. Consult the data system manufacturer's instructions for the proper sequence of operations. Calculations should be performed in a manner similar to that described in CALCULATIONS below in order to provide the desired result.

Sample Analysis Procedure

1) Remove the lid from the sample jar and add 10 ml of the extraction solvent/internal standard solution using the dispensing flask. Replace the cap and swirl the contents to insure that the tape 700 is not adhering to the sides of the jar and is totally submersed in solvent. Repeat for all samples.

2) Allow the samples to sit 16 hours (typically done overnight).

3) Swirl the contents of the jar to mix. Using a transfer pipette, transfer an aliquot of the sample extract to a properly labeled autosampler vial. Cap the vial. Replace jar lid and retain until analyses are complete. Repeat for all samples.

4) Place the vials in the autosampler in random order and start the analyses using the GC conditions described above. The first vial should be a dichloromethane blank. Several "check" standards should be placed (about every 20th sample) throughout the run to verify correct operation.

5) At the completion of the run, check each chromatogram to insure proper analysis. If a problem is suspected, trouble shoot and correct. Reanalyze samples as needed.

Calculations

The total micrograms of stearyl alcohol in each sample extract is calculated based on the relative response of the stearyl alcohol peak to that of the 1-hexadecanol internal standard. The ratio of the peak areas is multiplied by the relative response factor (determined at time of instrument calibration) and the micrograms of internal standard in the extract to yield the total μg of stearyl alcohol in a sample.

Instrument Calibration

Determine the instrumental relative response factor for the stearyl alcohol and the internal standard based on the areas of the stearyl alcohol and 1-hexadecanol peaks in the calibration standard chromatogram.

$$\text{Response factor (Rf)} = \frac{\text{Area}_{\text{Inst}}}{\text{weight}_{\text{Inst}}} \text{ X } \frac{\text{weight}_{\text{sa}}}{\text{Area}_{\text{sa}}} \text{ X } 10$$

where

$\text{Area}_{\text{inst}}$ GC peak area for the internal standard

$\text{Area}_{\text{sa}}$ GC peak area for the stearyl alcohol

$\text{weight}_{\text{inst}}$ micrograms of the internal standard used to prepare internal standard/extraction solvent

$\text{weight}_{\text{sa}}$ micrograms of the stearyl alcohol used to prepare the calibration standard

Sample Calculations

Calculate the total micrograms of stearyl alcohol in each sample using the peak areas from the sample chromatogram in the following equation:

$$\text{Total } \mu\text{g SA} = \frac{\text{Area}_{\text{sa}}}{\text{Area}_{\text{Inst}}} \text{ X Rf X } \frac{\text{weight}_{\text{Inst}}}{100}$$

where

$\text{Area}_{\text{inst}}$ GC peak area for the internal standard

$\text{Area}_{\text{as}}$ GC peak area for the stearyl alcohol

$\text{weight}_{\text{inst}}$ micrograms of the internal standard used to prepare internal standard/extraction solvent

Report amount of skin care composition transferred in $mg/cm^2$ where:

$$\text{Composition Transferred} = \frac{0.001 \text{ X } \mu\text{g of stearyl alcohol}}{\text{(concentration of stearyl alcohol in composition) X (tape area)}}$$

For the method described above the concentration of stearyl alcohol in the composition is 41 % and the tape patch measures 4.4 cm X 4.4 cm.

$$\text{Composition Transferred} = (0.001 \text{ X } \mu\text{g of stearyl alcohol})/(0.41 \text{ X } 4.4 \text{ cm X } 4.4 \text{ cm})$$

$$0.000126 \text{ X } \mu\text{g of stearyl alcohol (mg/cm}^2\text{)}$$

VII. Specific Examples

The following are specific illustrations which: a) demonstrate the method of determining chemically suitable proton donating actives; b) demonstrate preparation of various embodiments of the present invention; and c) demonstrate the efficacy of the present invention in helping maintain a wearer's skin at an acidic pH.

Example I

Skin pH Reduction Capability

This example is intended to demonstrate the pH reduction capability of several exemplary proton donating actives. Table 1 lists examples of potentially suitable proton donating actives for use in the present invention, baseline skin pH, and the measured pH after each of the ingredients was evaluated according to the Skin pH Reduction Test that is described in the TEST METHODS section.

Table 1

| Component | Baseline Skin pH | Post Application Skin pH | ΔpH |
|---|---|---|---|
| Control Lotion (No proton donating active) | 5.99±0.1 | 5.97±0.2 | 0.2±0.1 |
| 5% Citric Acid* | 5.72±0.1 | 4.17±0.4 | 1.55±.3 |
| 10.8% $NaH_2PO_4 \cdot H_2O$* | 6.01±0.1 | 5.05±0.2 | 0.96±0.2 |
| 5.6% Poly acrylic Acid[1] (~5,000 MW) | 5.92±0.1 | 3.32±0.3. | 2.60±0.3 |
| 5.6% Polyacrylic Acid[2] (~1,250,000 MW) | 5.69±0.1 | 3.35±0.2 | 2.33±0.3 |

1 Available from Aldrich Chemical Co., Inc. of Milwaukee, WI as Catalog Number 19203-1
2 Available from Aldrich of Chemical Co., Inc. of Milwaukee, WI as Catalog Number 30621-5
* Not falling under claim 1

All ΔpH values for lotions containing a potential proton donating active ingredient are significantly different from the ΔpH value for the control lotion at 95% confidence. These results clearly indicate that all of the materials tested in this example are capable of causing a reduction in skin pH.

Example 2 (not falling under claim 1)

Preparation of an Absorbent Article Having a Topsheet Comprising a Skin Care Composition

A. Preparation of Skin Care Composition

An exemplary skin care composition (Composition A) not falling under claim 1 of the present invention having a suspended proton donating active has the composition shown in Table 2 below:

Table 2

| Component | Weight % |
|---|---|
| Petrolatum[1] | 55 |
| Stearyl Alcohol[2] | 39 |
| Citric Acid | 5 |
| Aloe Vera Extract[3] | 1 |

1. Available from Witco Corp., Greenwich, CT as White Protopet®
2. Available from The Procter & Gamble Company, Cincinnati, OH as CO1897
3. Available from Madis Botanicals, Inc., South Hackensack, NJ as Veragel Lipoid in Kaydol

The composition may be prepared by melting (heat to a temperature of about 77°C) and mixing the petrolatum and the stearyl alcohol. The citric acid and the aloe may then be added to the melted mixture with further mixing to complete preparation of the composition.

B. Preparation of a Treated Article by Contact Slot Coating

Composition A is placed into a heated tank operating at a temperature of about 77°C. The composition is subsequently applied with a contact applicator (using, for example, a Meltex EP45 hot melt adhesive applicator head having 5 slots and operating at a temperature of about 77°C) onto the topsheet of an article in a striped pattern where the stripes run in the article's longitudinal direction. Specifically, 5 stripes are applied, each stripe measuring 0.25 in. wide (i.e., in the articles lateral direction) and 11.75 in. long at an add-on level = 7.7 mg/in$^2$ (12 g/m$^2$, 1.19 mg/cm$^2$). The distance between the stripes is 0.31 in.

The article to which skin care composition is added in this example is commercially available Pampers Premium (Size 4) diapers, available from Procter & Gamble, Cincinnati, OH.

Example 3

This example is intended to demonstrate the utility of skin care compositions comprising various proton donating actives in helping maintain an acidic skin pH for an extended period of time. Test skin care compositions as listed in Table 3 were prepared substantially as described in Example 2 with various proton donating actives. The skin care compositions were tested according to the Skin pH Reduction test described in the TEST METHODS section with the following exception. 0.1 milligrams per square centimeter were applied to subject forearms instead of 20 milligrams and there was no wiping step. Table 3 lists the results of skin pH reduction duration measurements made for a period of up to three hours (180 minutes) after application.

Table 3

| Test Skin Care Composition | Time Since Application | Measured Skin pH |
|---|---|---|
| Control composition with no Proton Donating Active | Baseline (Before Application) | 5.3±0.3 |
| | 1 Minute After Application | 5.3±0.3 |
| | 30 Minutes After Application | 5.2±0.2 |
| | 60 Minutes After Application | 5.3±0.3 |
| | 180 Minutes After Application | 5.1±0.2 |
| Composition A Prepared According to Example 2 | Baseline pH (Before Application) | 5.4±0.3 |
| | 1 Minute After Application | 3.6±0.4 |
| | 30 Minutes After Application | 3.5±0.3 |
| | 60 Minutes After Application | 3.5±0.3 |
| | 180 Minutes After Application | 3.7±0.3 |
| Skin Care Composition Prepared According to Example 2 with 5.6% Polyacrylic Acid (5000 MW) Replacing the Citric Acid | Baseline pH (Before Application) | 5.3±0.3 |
| | 1 Minute After Application | 3.2±0.1 |
| | 30 Minutes After Application | 3.6±0.1 |
| | 60 Minutes After Application | 3.6±0.1 |
| | 180 Minutes After Application | 3.7±0.1 |

All after-application skin pHs for compositions comprising a proton donating active are significantly (95% confidence) lower than the skin pH of the control composition at the same timepoint. This example clearly demonstrates the residual pH reduction benefit of the skin care compositions of the present invention.

Example 4 (not falling under claim 1)

This example is intended to demonstrate the transfer of the preferred skin care composition of the present invention from diapers prepared according to Example 2 to a wearer's skin.

Lotion transfer was measured after various wear times using the method described in the TEST METHODS section above. The results are given in Table 4.

Table 4

| *In vivo* Lotion Transfer | |
|---|---|
| Wear Time | Amount of Lotion Transferred (mg/$cm^2$) |
| 3 Hours | 0.05 |
| 6 Hours | 0.07 |
| 18 Hours | 0.10 |
| 24 Hours | 0.17 |

As can be seen, measurable amounts of the skin care composition transfer from the diaper to a wearer's skin. The Applicants have found that applying such amounts of a skin care composition comprising a proton donating active provides a meaningful reduction in skin pH.

Example 5 (not falling under claim 1)

This example is intended to demonstrate the preparation of an anhydrous skin care composition wherein the proton donating active is dissolved in the composition.

Table 5

| Component | Weight % |
|---|---|
| Propylene Glycol | 10 |
| Ceteareth-10[1] | 10 |
| Citric Acid | 20 |
| Cetearyl Alcohol[2] | 25 |
| Petrolatum | 35 |
| 1. Available from BASF Corporation, Mt. Olive, NJ<br>2. Available from the Procter & Gamble Company, Cincinnati, OH as TA1618 | |

The skin care may be prepared using a process comprising the following steps: 1) melt the ceteareth-10 by heating it to a temperature of between about 65°C and about 85°C; 2) add the propylene glycol and mix to provide a homogeneous solution; 3) add the cetearyl alcohol and mix to obtain a homogeneous solution while maintaining the temperature between about 65°C and about 85°C; 4) add the citric acid and mix until a clear solution is obtained while maintaining the temperature between about 65°C and about 85°C; and 5) add the petrolatum and mix to obtain a homogeneous solution while maintaining the temperature between about 65°C and about 85°C. This melted skin care composition may then be applied to an absorbent article as described in Example 2 above.

Example 6 (not falling under claim 1)

This example is intended to demonstrate how certain esters can be a source of a proton active ingredient in skin care compositions of the present invention.
The following three compositions were prepared:

1. Water + 50% triacetin.

2. Heated* Feces (1:2 w/w)+ 50% triacetin

3. Feces (1 :2 w/w) + 50% triacetin.

Compositions 1 and 2 are control compositions where there is no expected lipase activity. Composition 3 is expected to have lipase activity. The pH of each composition was measured immediately after mixing and after 24 hours of

incubation at 37 °C. Table 6 lists the results of this experiment.

Table 6

| Composition | Initial pH | Final pH | Δ pH |
|---|---|---|---|
| 1 | 4.2 | 4.1 | -0.1 |
| 2 | 6.6 | 6.4 | -0.2 |
| 3 | 5.8 | 4.5 | -1.3 |

These results clearly demonstrate that certain esters can be a source of proton donating actives that are effective in reducing the pH in the environment of a wearer's skin.

* Sufficient heat treatment to inactivate fecal enzymes

## Claims

**1.** An absorbent article for absorption of bodily fluids deposited by a wearer and for helping maintain said wearer's skin pH at an acidic level, said absorbent article comprising:

a. a body contacting surface having a skin care composition disposed on at least a portion thereof, **characterized in that**:

i. said skin care composition is transferable from said body contacting surface to said wearer's skin by contact, normal wearer motion and/or body heat,
ii. said skin care composition comprises at least one proton donating active selected from the group consisting of polyacrylic acid and partially neutralized polyacrylic acid, and
iii. said skin care composition has a continuous phase comprising substantially oleaginous materials;

b. a liquid impermeable backsheet; and
c. an absorbent core positioned between said body contacting surface and said backsheet.

**2.** An absorbent article according to Claim 1 wherein said skin care composition comprises at least 0.5% of said proton donating active, preferably between 0.5% and 20% of said proton donating active, more preferably between 3% and 7% proton donating active.

**3.** An absorbent article according to Claim 1 wherein said proton donating active has at least one pKa between 2.0 and 6.5, preferably between 2.5 and 5.0.

**4.** An absorbent article according to Claim any of the above claims wherein said skin care composition comprises an anhydrous composition.

**5.** An absorbent article according to Claim 4 wherein said anhydrous composition comprises a mixture of an emollient and an immobilizing agent, said emollient preferably being selected from the group consisting of: petroleum-based emollients, sucrose esters of fatty acids, fatty acid esters, humectants, lanolin, lanolin derivatives, polysiloxane emollients, and mixtures thereof and said immobilizing agent preferably being selected from the group consisting of $C_{14}$-$C_{22}$ fatty alcohols, $C_{12}$-$C_{22}$ fatty acids, and C $_{12}$-$C_{22}$ fatty alcohol ethoxylates having an average degree of ethoxylation ranging from 2 to 30, waxes, including microcryatalline waxes, and mixtures thereof.

**6.** An absorbent article according to Claims 4 or 5 wherein said proton donating active is dissolved in the remainder of said anhydrous composition.

**7.** An absorbent article according to Claim 1 wherein said body contacting surface is selected from the group consisting of a topsheet, an elastic leg cuff, an elastic waist feature, and a side panel.

## Patentansprüche

1. Absorptionsartikel zur Absorption von Körperflüssigkeiten, die von einem Träger ausgeschieden werden, und zur Unterstützung, dass der pH-Wert der Haut des Trägers auf einem saueren Niveau bleibt, wobei der Absorptionsartikel Folgendes umfasst:

   a. eine den Körper berührende Oberfläche mit einer Hautpflegezusammensetzung, die auf mindestens einem Teil davon aufgebracht ist, **dadurch gekennzeichnet, dass**:

      i. die Hautpflegezusammensetzung von der den Körper berührenden Oberfläche durch Kontakt, normale Trägerbewegung und/oder Körperwärme auf die Haut des Trägers übertragen werden kann,
      ii. die Hautpflegezusammensetzung mindestens einen protonengebenden Wirkstoff umfasst, der ausgewählt ist aus der Gruppe, bestehend aus Polyacrylsäure und teilweise neutralisierter Polyacrylsäure, und
      iii. die Hautpflegezusammensetzung eine kontinuierliche Phase auf weist, die im Wesentlichen ölartige Materialien umfasst;

   b. eine flüssigkeitsundurchlässige Unterschicht; und
   c. einen Absorptionskern, der zwischen der den Körper berührenden Oberfläche und der Unterschicht angeordnet ist.

2. Absorptionsartikel nach Anspruch 1, wobei die Hautpflegezusammensetzung zu mindestens 0,5 % den protonengebenden Wirkstoff, vorzugsweise zwischen 0,5 % und 20 % den protonengebenden Wirkstoff, mehr bevorzugt zwischen 3 % und 7 % den protonengebenden Wirkstoff umfasst.

3. Absorptionsartikel nach Anspruch 1, wobei der protonengebende Wirkstoff mindestens einen pKa zwischen 2,0 und 6,5, vorzugsweise zwischen 2,5 und 5,0 aufweist.

4. Absorptionsartikel nach einem der vorstehenden Ansprüche, wobei die Hautpflegezusammensetzung eine wasserfreie Zusammensetzung umfasst.

5. Absorptionsartikel nach Anspruch 4, wobei die wasserfreie Zusammensetzung eine Mischung aus einem Weichmacher und einem Immobilisierungsmittel umfasst, wobei der Weichmacher vorzugsweise ausgewählt ist aus der Gruppe, bestehend aus: Weichmachern auf Erdölbasis, Saccharoseestern von Fettsäuren, Fettsäureestern, Feuchthaltemitteln, Lanolin, Lanolinderivaten, Polysiloxan-Weichmachern und Mischungen davon, und das Immobilisierungsmittel vorzugsweise ausgewählt ist aus der Gruppe, bestehend aus $C_{14}$-$C_{22}$-Fettalkoholen, $C_{12}$-$C_{22}$-Fettsäuren und $C_{12}$-$C_{22}$-Fettalkoholethoxylaten mit einem durchschnittlichen Ethoxylierungsgrad im Bereich von 2 bis 30, Wachsen, einschließlich mikrokristallinen Wachsen, und Mischungen davon.

6. Absorptionsartikel nach Anspruch 4 oder 5, wobei der protonengebende Wirkstoff im übrigen Teil der wasserfreien Zusammensetzung gelöst wird.

7. Absorptionsartikel nach Anspruch 1, wobei die den Körper berührende Oberfläche ausgewählt ist aus der Gruppe, bestehend aus einer Oberschicht, einem elastischen Beinbündchen, einem elastischen Taillenelement und einem Seitenfeld.

## Revendications

1. Article absorbant pour l'absorption de fluides corporels déposés par un porteur et pour aider à maintenir le pH de la peau dudit porteur à un niveau acide, ledit article absorbant comprenant :

   a. une surface en contact avec le corps ayant une composition de soin de la peau disposée sur au moins une partie de celle-ci, **caractérisée en ce que** :

      i. ladite composition de soin de la peau peut être transférée de ladite surface en contact avec le corps sur ladite peau du porteur par contact, mouvement normal du porteur et/ou chaleur du corps,
      ii. ladite composition de soin de la peau comprend au moins un principe actif donneur de proton choisi dans le groupe constitué d'acide polyacrylique et d'acide polyacrylique partiellement neutralisé, et iii. ladite composition de soin de la peau a une phase continue comprenant des matériaux essentiellement oléagineux ;

b. une feuille de fond imperméable aux liquides ; et
c. une âme absorbante positionnée entre ladite surface en contact avec le corps et ladite feuille de fond.

**2.** Article absorbant selon la revendication 1, dans lequel ladite composition de soin de la peau comprend au moins 0,5 % dudit principe actif donneur de proton, de préférence entre 0,5 % et 20 % dudit principe actif donneur de proton, plus préférablement entre 3 % et 7 % de principe actif donneur de proton.

**3.** Article absorbant selon la revendication 1, dans lequel ledit principe actif donneur de proton a au moins un pKa compris entre 2,0 et 6,5, de préférence entre 2,5 et 5,0.

**4.** Article absorbant selon l'une quelconque des revendications précédentes, dans lequel ladite composition de soin de la peau comprend une composition anhydre.

**5.** Article absorbant selon la revendication 4, dans lequel ladite composition anhydre comprend un mélange d'un émollient et d'un agent immobilisant, ledit émollient étant choisi de préférence dans le groupe constitué de : émollients à base de pétrole, esters de saccharose d'acides gras, esters d'acide gras, humectants, lanoline, dérivés de lanoline, émollients de type polysiloxane, et leurs mélanges et ledit agent immobilisant étant choisi de préférence dans le groupe constitué d'alcools gras en $C_{14}$ à $C_{22}$, acides gras en $C_{12}$ à $C_{22}$ et éthoxylates d'alcool gras en $C_{12}$ à $C_{22}$ ayant un degré moyen d'éthoxylation allant de 2 à 30, cires, y compris des cires microcristallines, et leurs mélanges.

**6.** Article absorbant selon les revendications 4 ou 5, dans lequel ledit principe actif donneur de proton est dissous dans le reste de ladite composition anhydre.

**7.** Article absorbant selon la revendication 1, dans lequel ladite surface en contact avec le corps est choisie dans le groupe constitué d'une feuille de dessus, un revers de jambe élastique, un élément de ceinture élastique et un pan latéral.

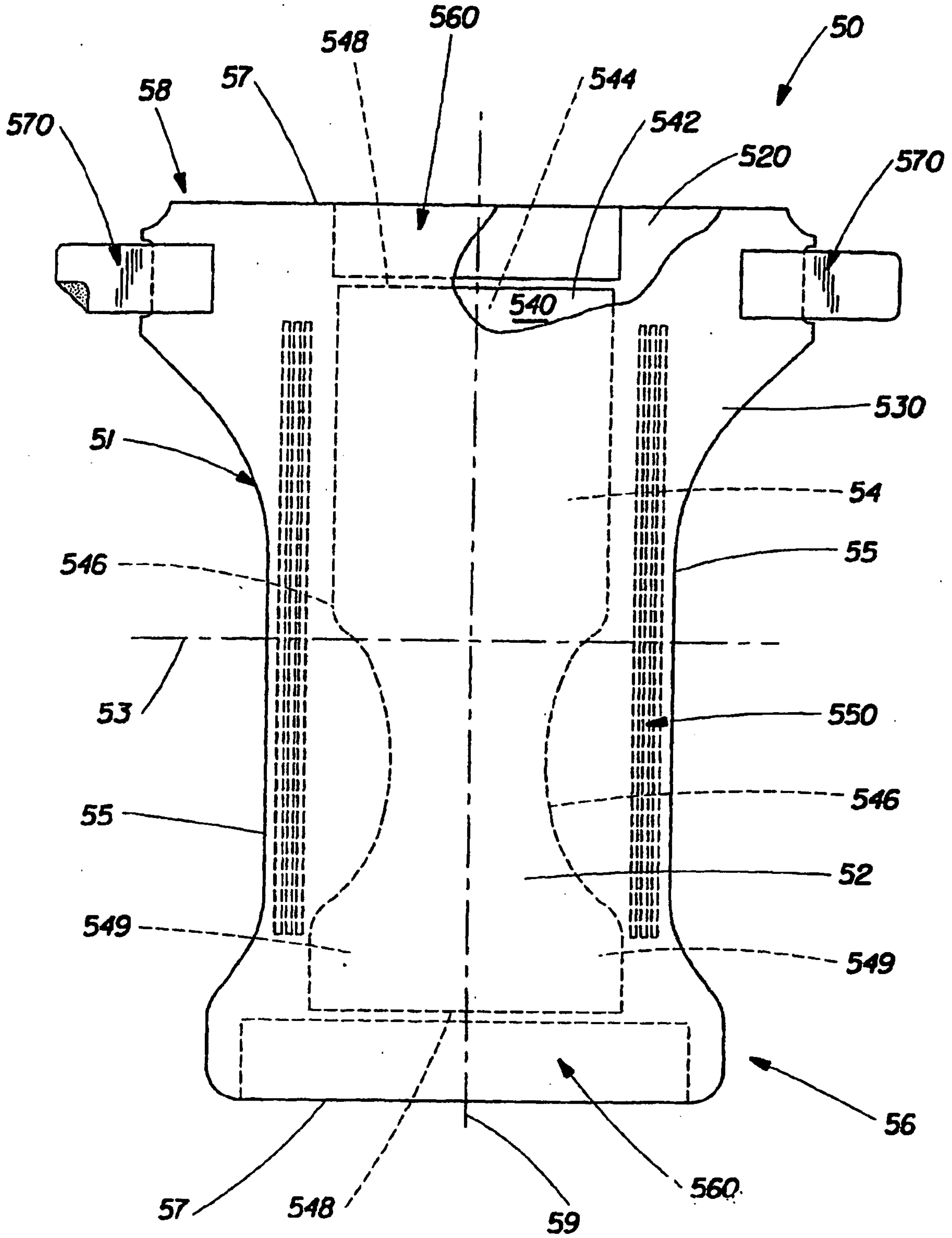
50
560
548
544
542
520
570
58
57
570
540
530
51
54
55
546
53
550
546
55
52
549
549
56
57
548
59
560

Fig. 1

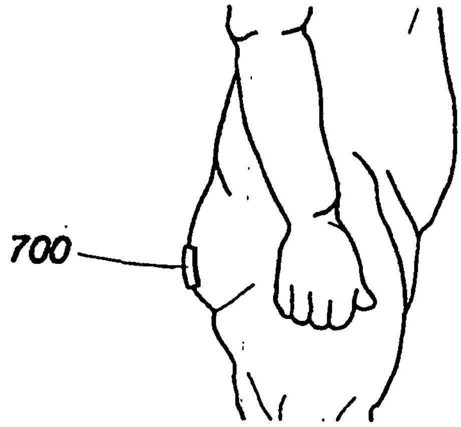


Fig. 2A

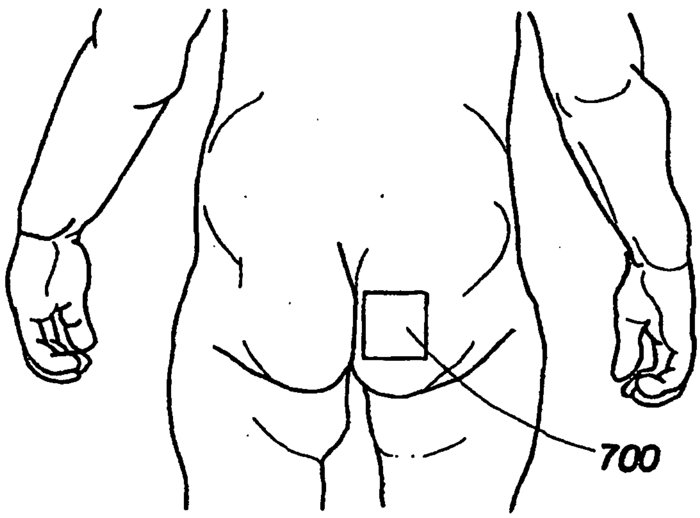


Fig. 2B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3707148 A
- US 3964486 A
- US 4685909 A
- US 5525346 A
- US 4573986 A
- US 3911173 A
- US 4785996 A
- US 4842666 A
- US 3860003 A
- US 5151092 A
- US 5221274 A
- US 5554145 A
- US 4988344 A
- US 4988345 A
- US 3929135 A
- US 4324246 A
- US 4342314 A
- US 4463045 A
- US 5006394 A
- US 4609518 A
- US 4629643 A
- US 4950264 A
- US 4610678 A
- US 4673402 A
- US 4888231 A
- US 4834735 A
- US 5234423 A
- US 5147345 A
- US 4909803 A
- US 4695278 A
- US 4795454 A
- US 766386 A
- US 08840039 B
- US 4515595 A
- US 5026364 A
- US 4857067 A
- US 4381781 A
- US 4938753 A
- US 4846815 A
- US 4894060 A
- US 4946527 A
- US 3848594 A
- US 4662875 B1
- US 4892536 A
- US 4990147 A, Freeland
- US 07993198 A
- US 5171236 A
- US 5246433 A
- US 5569234 A
- US 4940464 A
- US 5092861 A
- US 4253461 A
- US 4597760 A
- US 4597761 A
- US 4704115 A
- US 4909802 A
- US 4964860 A
- US 5304161 A
- US 4556146 A
- US 4589876 B1
- US 4687478 A
- US 5009653 A
- US 5267992 A
- US 5389094 A
- US 5413568 A
- US 5460623 A
- US 5489283 A
- US 5569231 A
- US 5620430 A
- US 5643588 A
- US 926532 A
- US 08926533 B
- US 5607760 A
- US 5609587 A
- US 5635191 A
- US 5059282 A
- US 5174927 A
- US 5624676 A
- US 4011389 A